(19) Europäisches Patentamt
European Patent Office
Office européen des brevets

(11) EP 4 506 370 A1

(12) EUROPEAN PATENT APPLICATION
published in accordance with Art. 153(4) EPC

(43) Date of publication:
**12.02.2025 Bulletin 2025/07**

(21) Application number: **23784668.8**

(22) Date of filing: **27.03.2023**

(51) International Patent Classification (IPC):
***C08F 2/38*** *(2006.01)* ***C08F 20/18*** *(2006.01)*

(52) Cooperative Patent Classification (CPC):
**C07C 69/618; C07C 255/41; C08F 2/38;**
C08F 220/1804

(86) International application number:
**PCT/JP2023/012338**

(87) International publication number:
**WO 2023/195378 (12.10.2023 Gazette 2023/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.04.2022 JP 2022063295**
**09.03.2023 JP 2023036552**

(71) Applicants:
- **Shinshu University**
  **Matsumoto-shi, Nagano 390-8621 (JP)**
- **Soken Chemical & Engineering Co., Ltd.**
  **Toshima-ku**
  **Tokyo 171-8531 (JP)**

(72) Inventors:
- **KOHSAKA, Yasuhiro**
  **Ueda City, Nagano 3868567 (JP)**
- **KAWATANI, Ryo**
  **Ueda City, Nagano 3868567 (JP)**
- **OKAMOTO, Syuji**
  **Tokyo 171-8531 (JP)**
- **MATSUZAKI, Hironori**
  **Tokyo 171-8531 (JP)**
- **YOSHINO, Mizuki**
  **Tokyo 171-8531 (JP)**

(74) Representative: **Gulde & Partner**
**Patent- und Rechtsanwaltskanzlei mbB**
**Berliner Freiheit 2**
**10785 Berlin (DE)**

# (54) CHAIN TRANSFER AGENT, PRODUCTION METHOD OF POLYMER, POLYMER, PRESSURE-SENSITIVE ADHESIVE COMPOSITION AND POLYMER PARTICLES

(57) The present invention aims to provide a chain transfer agent other than a mercaptan compound, which can sufficiently control the molecular weight.

According to the present invention, provided is a chain transfer agent represented by following general formula (1), wherein:

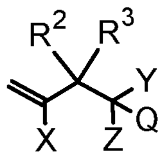
……(1)

in general formula (1), X is a group selected from among a cyano group, an optionally substituted aryl group, an acyl group, a carboxy group, $COOR^1$, $C(O)NH_2$, $C(O)NHR^1$, $C(O)NR^1_2$, $SOR^1$, $SO_2R^1$, $SO_3R^1$, and $OR^1$; each Y and Z is a group selected from among a cyano group, an optionally substituted aryl group, an acyl group, a carboxy group, $COOR^1$, $C(O)NH_2$, $C(O)NHR^1$, $C(O)NR^1_2$, $SOR^1$, $SO_2R^1$, $SO_3R^1$, $OR^1$, $OC(O)R^1$, $N(H)C(O)R^1$, and a halogen, or Y and Z are bonded to form an optionally substituted carbocyclic structure or an optionally substituted heterocyclic structure; X, Y and Z are the same or different; $R^1$ is an optionally substituted alkyl group having 1 to 8 carbon atoms;
Q is hydrogen, an optionally substituted alkyl group having 1 to 6 carbon atoms or a phenyl group,
$R^2$ and $R^3$ are groups selected from hydrogen, an alkyl group having 2 to 6 carbon atoms, an optionally substituted aryl group, and an optionally substituted heterocyclic structure, and $R^2$ and $R^3$ are the same or different, and general formula (1) excludes a case where both $R^2$ and $R^3$ are hydrogen.

EP 4 506 370 A1

# Description

## Technical Field

The present invention relates to a chain transfer agent, a production method of a polymer, a polymer, an adhesive composition, and a polymer particle.

## Background Art

(First perspective)

When a radically polymerizable compound such as methyl methacrylate or the like is polymerized, a chain transfer agent is used for the purpose of controlling the molecular weight of the polymer or the purpose of the like, and mercaptan compounds such as n-dodecyl mercaptan have been used conventionally.
Patent Literature 1 discloses a chain transfer agent consisting of a compound having a maleimide group and a mercapto group. Patent Literature 2 discloses a thiol compound having a specific structure, and discloses the use of the compound as a chain transfer agent.

(Second perspective)

In addition, many techniques have been disclosed for polymerizing polymers for various applications using sulfur-containing thiol-based chain transfer agents such as n-dodecyl mercaptan, and the like. For example, Patent Literature 3 discloses a method for producing (meth)acrylic crosslinked particles, comprising a step of polymerizing a monomer component containing a (meth)acrylic acid alkyl ester and a (meth)acrylic-based polyfunctional monomer in the presence of a thiol-based chain transfer agent.

## Citation List

### Patent Literature

Patent Literature 1: JP-A-2003-321506
Patent Literature 2: JP-A-2004-292428
Patent Literature 3: WO2017/022423

## Summary of Invention

### Technical Problem

(First perspective)

Mercaptan compounds have the advantage of being able to efficiently control the molecular weight of polymers. On the other hand, mercaptan compounds have problems such as having peculiar odors and polymers polymerized using mercaptan compounds as chain transfer agents are not preferred for use depending on the area of application. Chain transfer agents other than mercaptan compounds have also been developed, but these have not been able to sufficiently control the molecular weight.
The present invention has been made by taking these circumstances into consideration, and aims to provide a chain transfer agent other than a mercaptan compound, which is capable of sufficiently controlling the molecular weight.

(Second perspective)

Mercaptan compounds have the advantage of being able to efficiently control the molecular weight of polymers. On the other hand, mercaptan compounds have problems such as peculiar odors, and depending on the area of application, polymers polymerized using mercaptan compounds as chain transfer agents can cause corrosion.
The present invention has been made by taking these circumstances into consideration, and aims to provide a polymer which is less likely to cause corrosion problems, as compared with polymers produced using conventional thiol-based chain transfer agents such as mercaptan compounds or the like, and also to provide a method for producing a

polymer which is less likely to cause corrosion problems, as compared with the case of using conventional thiol-based chain transfer agents such as mercaptan compounds or the like, and which allows sufficient control of the molecular weight.

## Solution to Problem

(First perspective)

According to the present invention, provided is a chain transfer agent represented by following general formula (1).

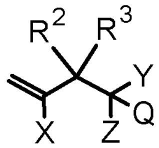

(1)

In general formula (1), X is a group selected from among a cyano group, an optionally substituted aryl group, an acyl group, a carboxy group, $COOR^1$, $C(O)NH_2$, $C(O)NHR^1$, $C(O)NR^1_2$, $SOR^1$, $SO_2R^1$, $SO_3R^1$, and $OR^1$; each Y and Z is a group selected from among a cyano group, an optionally substituted aryl group, an acyl group, a carboxy group, $COOR^1$, $C(O)NH_2$, $C(O)NHR^1$, $C(O)NR^1_2$, $SOR^1$, $SO_2R^1$, $SO_3R^1$, $OR^1$, $OC(O)R^1$, $N(H)C(O)R^1$, and a halogen, or Y and Z are bonded to form an optionally substituted carbocyclic structure or an optionally substituted heterocyclic structure; X, Y and Z are the same or different; $R^1$ is an optionally substituted alkyl group having 1 to 8 carbon atoms; Q is hydrogen, an optionally substituted alkyl group having 1 to 6 carbon atoms or a phenyl group; $R^2$ and $R^3$ are groups selected from among hydrogen, an alkyl group having 2 to 6 carbon atoms, an optionally substituted aryl group, and an optionally substituted heterocyclic structure. $R^2$ and $R^3$ are the same or different, and general formula (1) excludes a case where both $R^2$ and $R^3$ are hydrogen.

The present inventors have conducted intensive research, and have found that when a compound represented by the above general formula (1) is used as a chain transfer agent to polymerize a monomer to obtain a polymer, the molecular weight of the obtained polymer can be sufficiently controlled, thereby leading to completion of the present invention.

(Second perspective)

According to the present invention, provided is a polymer containing a polymer chain having a group represented by following general formula (18) at an end thereof.

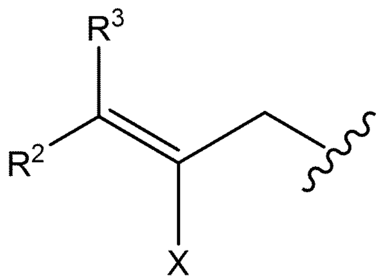

(18)

In general formula (18), X is a group selected from among a cyano group, an optionally substituted aryl group, an acyl group, a carboxy group, $COOR^1$, $C(O)NH_2$, $C(O)NHR^1$,$C(O)NR^1_2$, $SOR^1$, $SO_2R^1$, $SO_3R^1$, and $OR^1$; $R^1$ is an optionally substituted alkyl group having 1 to 8 carbon atoms;

$R^2$ and $R^3$ are groups selected from among hydrogen, an alkyl group having 2 to 6 carbon atoms, an optionally substituted aryl group, and an optionally substituted heterocyclic structure; $R^2$ and $R^3$ are the same or different, and general formula (18) excludes a case where both $R^2$ and $R^3$ are hydrogen.

The present inventors have conducted intensive research, and have found that when a polymer is obtained by polymerizing a monomer using a compound having a specific structure as a chain transfer agent, it is possible to obtain a polymer which is less likely to cause a corrosion problem compared to a polymer obtained using a conventional thiol-based chain transfer agent such as a mercaptan compound or the like, and also possible to sufficiently control the molecular weight, thereby leading to completion of the present invention.

Various embodiments of the present invention will be exemplified below. The embodiments shown below can be combined with each other.

[1] A chain transfer agent represented by following general formula (1), wherein:

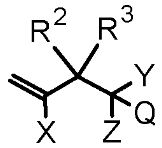

(1)

in general formula (1), X is a group selected from among a cyano group, an optionally substituted aryl group, an acyl group, a carboxy group, $COOR^1$, $C(O)NH_2$, $C(O)NHR^1$, $C(O)NR^1{}_2$, $SOR^1$, $SO_2R^1$, $SO_3R^1$, and $OR^1$; each Y and Z is a group selected from among a cyano group, an optionally substituted aryl group, an acyl group, a carboxy group, $COOR^1$, $C(O)NH_2$, $C(O)NHR^1$, $C(O)NR^1{}_2$, $SOR^1$, $SO_2R^1$, $SO_3R^1$, $OR^1$, $OC(O)R^1$, $N(H)C(O)R^1$, and a halogen, or Y and Z are bonded to form an optionally substituted carbocyclic structure or an optionally substituted heterocyclic structure; X, Y and Z are the same or different; $R^1$ is an optionally substituted alkyl group having 1 to 8 carbon atoms; Q is hydrogen, an optionally substituted alkyl group having 1 to 6 carbon atoms or a phenyl group, $R^2$ and $R^3$ are groups selected from among hydrogen, an alkyl group having 2 to 6 carbon atoms, an optionally substituted aryl group, and an optionally substituted heterocyclic structure, and $R^2$ and $R^3$ are the same or different, and general formula (1) excludes a case where both $R^2$ and $R^3$ are hydrogen.

[2] The chain transfer agent of [1], wherein at least one of $R^2$ and $R^3$ is hydrogen.

[3] The chain transfer agent of [1] or [2], wherein at least one of $R^2$ and $R^3$ is a group selected from among an optionally substituted aryl group and an optionally substituted heterocyclic structure.

[4] The chain transfer agent of any one of [1] to [3], wherein the chain transfer agent comprises the skeleton represented by general formula (23), wherein:

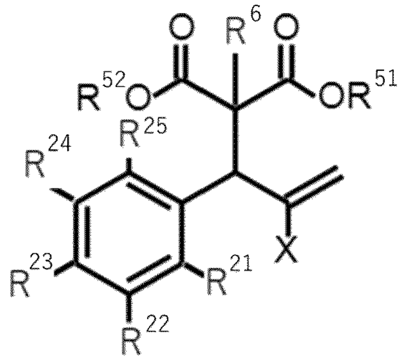

(23)

in general formula (23), $R^{21}$, $R^{22}$, $R^{23}$, $R^{24}$, and $R^{25}$ are groups selected from among hydrogen, an alkyl group having 1 to 8 carbon atoms, an alkoxy group having 1 to 8 carbon atoms, a cyano group, a carboxy group, and a halogen; $R^{21}$, $R^{22}$, $R^{23}$, $R^{24}$, and $R^{25}$ are the same or different;

X is a group selected from among a cyano group, an optionally substituted aryl group, an acyl group, a carboxy group, $COOR^1$, $C(O)NH_2$, $C(O)NHR^1$, $C(O)NR^1{}_2$, $SOR^1$, $SO_2R^1$, $SO_3R^1$, and $OR^1$; $R^1$ is an optionally substituted alkyl group having 1 to 8 carbon atoms;

$R^{51}$ and $R^{52}$ are alkyl groups having 1 to 8 carbon atoms; $R^{51}$ and $R^{52}$ are the same or different;

$R^6$ is hydrogen, an optionally substituted alkyl group having 1 to 6 carbon atoms, or a phenyl group.

[5] A production method of a polymer, comprising a polymerization step of polymerizing a radically polymerizable compound in the presence of the chain transfer agent of any one of [1] to [4].

[6] The production method of [5], wherein in the polymerization step, the radically polymerizable compound is polymerized in one or more of organic solvents selected from among ethyl acetate, ethyl methyl ketone, and toluene.

[7] A method for producing an adhesive composition, comprising a polymer production step and a crosslinking agent blending step, wherein in the polymer production step, a polymer is produced by the polymer production method described in [6], and in the crosslinking agent blending step, at least one crosslinking agent selected from among an isocyanate crosslinking agent, an epoxy crosslinking agent, and a metal chelate crosslinking agent, is blended with the produced polymer.

[8] The production method of [5], wherein in the polymerization step, the radically polymerizable compound is polymerized in an aqueous medium.

[9] A polymer containing a polymer chain having a group represented by following general formula (18) at an end thereof, wherein:

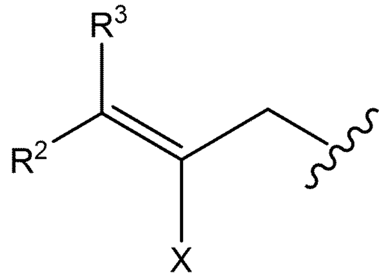
(18)

in general formula (18), X is a group selected from among a cyano group, an optionally substituted aryl group, an acyl group, a carboxy group, $COOR^1$, $C(O)NH_2$, $C(O)NHR^1$,$C(O)NR^1{}_2$, $SOR^1$, $SO_2R^1$, $SO_3R^1$, and $OR^1$; $R^1$ is an optionally substituted alkyl group having 1 to 8 carbon atoms; $R^2$ and $R^3$ are groups selected from among hydrogen, an alkyl group having 2 to 6 carbon atoms, an optionally substituted aryl group, and an optionally substituted heterocyclic structure; $R^2$ and $R^3$ are the same or different, and general formula (18) excludes a case where both $R^2$ and $R^3$ are hydrogen.

[10] The polymer of [9], wherein the group represented by the above general formula (18) is represented by following general formula (21), wherein:

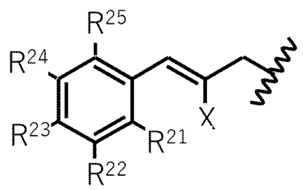
(21)

in general formula (21), $R^{21}$, $R^{22}$, $R^{23}$, $R^{24}$, and $R^{25}$ are groups selected from among hydrogen, an alkyl group having 1 to 8 carbon atoms, an alkoxy group having 1 to 8 carbon atoms, a cyano group, a carboxy group, and a halogen; $R^{21}$, $R^{22}$, $R^{23}$, $R^{24}$, and $R^{25}$ are the same or different; X is a group selected from among a cyano group, an optionally substituted aryl group, an acyl group, a carboxy group, $COOR^1$, $C(O)NH_2$, $C(O)NHR^1$,$C(O)NR^1{}_2$, $SOR^1$, $SO_2R^1$, $SO_3R^1$, and $OR^1$; $R^1$ is an optionally substituted alkyl group having 1 to 8 carbon atoms.

[11] The polymer of [9] or [10], wherein the polymer chain having a group represented by general formula (18) at an end thereof further has a group represented by following general formula (19) at an end thereof, wherein:

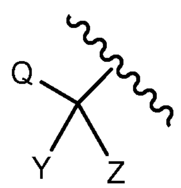
(19)

each Y and Z is a group selected from among a cyano group, an optionally substituted aryl group, an acyl group, a carboxy group, $COOR^1$, $C(O)NH_2$, $C(O)NHR^1$, $C(O)NR^1{}_2$, $SOR^1$, $SO_2R^1$, $SO_3R^1$, $OR^1$, $OC(O)R^1$, $N(H)C(O)R^1$, and a halogen, or Y and Z are bonded to form an optionally substituted carbocyclic structure or an optionally substituted heterocyclic structure; Y and Z are the same or different; $R^1$ is an optionally substituted alkyl group having 1 to 8 carbon atoms;

Q is hydrogen, an optionally substituted alkyl group having 1 to 6 carbon atoms, or a phenyl group.

[12] The polymer of [11], wherein the group represented by the above general formula (19) is represented by following general formula (22), wherein:

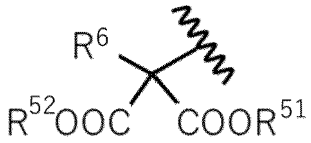

(22)

in general formula (22), $R^{51}$ and $R^{52}$ are alkyl groups having 1 to 8 carbon atoms; $R^{51}$ and $R^{52}$ are the same or different; $R^6$ is hydrogen, an optionally substituted alkyl group having 1 to 6 carbon atoms, or a phenyl group.

[13] The polymer of [9] or [10], wherein a content ratio of sulfur derived from a thiol-based chain transfer agent is 100 ppm or less.

[14] The polymer of [9] or [10], wherein the polymer chain has a repeating monomer unit, and the repeating monomer unit has at least one group selected from among a carboxy group, a hydroxyl group, an amino group, and an amide group.

[15] An adhesive composition comprising the polymer of [14] and at least one crosslinking agent selected from among an isocyanate crosslinking agent, an epoxy crosslinking agent, and a metal chelate crosslinking agent.

[16] A polymer particle comprising the polymer of [9] or [10], wherein the polymer particle has an average particle diameter of 0.05 to 500 μm.

## Effects of Invention

(First perspective)

When a polymer is produced by polymerizing a monomer using the chain transfer agent of the present invention, a polymer having a controlled molecular weight can be efficiently obtained. In addition, according to the production method of the present invention, a polymer having a sufficiently controlled molecular weight can be obtained. Furthermore, the chain transfer agent of the present invention has less odor compared to a chain transfer agent that is a mercaptan compound. Also, the polymer polymerized using the chain transfer agent of the present invention has no limited uses compared to a polymer polymerized using a mercaptan compound as a chain transfer agent.

(Second perspective)

According to the present invention, provided is a polymer which is less likely to cause corrosion problems as compared with a polymer produced using a conventional thiol-based chain transfer agent such as a mercaptan compound or the like, and also to provide a method for producing a polymer which causes less of the above problems as compared with the case of using a conventional thiol-based chain transfer agent such as a mercaptan compound or the like, and which is capable of sufficiently controlling the molecular weight.

## Brief Description of Drawings

Fig. 1 shows microscopic images of the polymer particles of Examples 2-10 and 2-12.

Fig. 2 shows microscopic images of the polymer particles of Comparative Example 2-4 and Reference Example 2-6.

## Description of Embodiments

Hereinafter, the present invention will be described in detail below by exemplifying the embodiments of the present invention. The present invention is not limited to these descriptions. The distinctive features of the embodiments of the present invention described below can be combined with each other. In addition, an invention can be established independently for each of the distinctive features.

1. Chain Transfer Agent

The chain transfer agent of the present invention has a structure represented by following general formula (1).

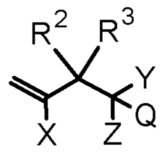

(1)

In general formula (1), X is a group selected from among a cyano group, an optionally substituted aryl group, an acyl group, a carboxy group, $COOR^1$, $C(O)NH_2$, $C(O)NHR^1$, $C(O)NR^1{}_2$, $SOR^1$, $SO_2R^1$, $SO_3R^1$, and $OR^1$.

The reaction mechanism when the compound represented by general formula (1) functions as a chain transfer agent for radical polymerization can be explained as follows. First, an addition reaction occurs in which the vinylidene group accepts the attack of radical species, followed by a cleavage reaction accompanied by recombination of the double bond and elimination of the methyl group substituted with the allyl substituent, i.e., Q, Y, and Z, to generate a methyl radical substituted with Q, Y, and Z. The series of the above reaction mechanisms is called an addition-cleavage reaction, in which X determines the ease of the addition reaction, and Y, Z, $R^2$, and $R^3$ determine the ease of the cleavage reaction. Preferably, an optimal X is selected according to the expected monomer. When a reinitiation reaction from the radical species freed by the chain transfer reaction is expected, preferably optimal Q, Y, and Z are selected according to the expected monomer. The forms of X, Y, Z, $R^2$, and $R^3$ and their operating principles are described below.

In one embodiment of the present invention, X can be a cyano group. When X is a cyano group, an induction effect of a nitrogen atom and a resonance effect of the cyano group and the vinylidene group work to create a bias (polarization) of electron density between the two carbon atoms comprising the vinylidene group. As a result, the vinylidene group is more likely to accept the addition of radical species. Furthermore, when a radical is added to the vinylidene group, a new radical species is generated, but the cyano group also has a function of delocalizing the unpaired electron of this radical by a resonance effect and stabilizing the radical species. In other words, compared with non-conjugated olefins in which X is a hydrogen atom or an alkyl group, radical addition is significantly more likely to occur when X is a cyano group.

In one embodiment of the present invention, X can be an optionally substituted aryl group. The aryl group may be a single ring, a condensed ring in which two or more aromatic rings are condensed, or a linked ring in which two or more aromatic rings are linked. The number of carbon atoms in the aromatic ring constituting the aryl group can be 6 to 18, preferably 6 to 12, and more preferably 6 to 10. Examples of the aryl group include a phenyl group, a 1-naphthyl group, a 2-naphthyl group, a 2-biphenyl group, a 3-biphenyl group, and a 4-biphenyl group, and the aryl group is preferably a phenyl group. The aryl group can have a substituent, and examples of the substituent include an alkyl group having 1 to 8 carbon atoms, a cyano group, and a carboxy group.

When X is an optionally substituted aryl group, a resonance effect of the aryl group and the vinylidene group works, and a bias (polarization) of the electron density occurs between the two carbon atoms constituting the vinylidene group. As a result, the vinylidene group is more likely to accept the addition of radical species. Furthermore, when a radical is added to the vinylidene group, a new radical species is generated, but the aryl group also has a function of delocalizing the unpaired electron of this radical by the resonance effect, stabilizing the radical species. In addition, when the aryl group is substituted with an alkyl group, a cyano group, or a carboxy group, a large resonance effect and a hyperconjugation effect including these substituents work, and the radical is further stabilized compared to an unsubstituted aryl group. From the above, compared with non-conjugated olefins in which X is a hydrogen atom or an alkyl group, radical addition is significantly more likely to occur when X is an optionally substituted aryl group.

In an embodiment of the present invention, X can be an acyl group ($C(=O)R^1$), a carboxy group, $COOR^1$, an amide group ($C(O)NH_2$, $C(O)NHR^1$, or $C(O)NR^1{}_2$), or a sulfone group-containing substituent $SOR^1$, $SO_2R^1$, or $SO_3R^1$. Examples of the acyl group include a formyl group, an acetyl group, a propionyl group, and a benzoyl group. Examples of $COOR^1$, that is, an alkoxycarbonyl group ($R^1OC(=O)$), include a methoxycarbonyl group, an ethoxycarbonyl group, and a tert-butoxycarbonyl group. Among the amide groups, examples of the N-substituted amide group ($C(O)NHR^1$ or $C(O)NR^1{}_2$) include an N-substituted amide group in which $R^1$ is a methyl group, an ethyl group, an n-propyl group, or an isopropyl group. When X is an acyl group ($C(=O)R^1$), a carboxy group, $COOR^1$, an amide group ($C(O)NH_2$, $C(O)NHR^1$, or $C(O)NR^1{}_2$), or a sulfone group-containing substituent $SOR^1$, $SO_2R^1$, or $SO_3R^1$, an inductive effect of the oxygen atom of the carbonyl/sulfone group and a resonance effect between the carbonyl/sulfone group and the vinylidene group work, causing a bias (polarization) of the electron density between the two carbon atoms constituting the vinylidene group. As a result, the vinylidene group becomes more likely to accept the addition of radical species. Furthermore, when a radical is added to the vinylidene group, a new radical species is generated, but the carbonyl/sulfone group also has a function of delocalizing the unpaired electron of this radical by the resonance effect, stabilizing the radical species. In other words, compared with non-conjugated olefins in which X is a hydrogen atom or an alkyl group, radical addition is significantly more likely to occur, when X is an acyl group ($C(=O)R^1$), a carboxy group, $COOR^1$, an amide group ($C(O)NH_2$, $C(O)NHR^1$, or $C(O)NR^1{}_2$), or a sulfone group-containing substituent $SOR^1$, $SO_2R^1$, or $SO_3R^1$.

In an embodiment of the present invention, X can be $OR^1$. When X is $OR^1$, an inductive effect of the oxygen atom and a resonance effect of the oxygen atom and the vinylidene group work, causing a bias (polarization) of the electron

density between the two carbon atoms constituting the vinylidene group. As a result, the vinylidene group is more likely to accept the addition of radical species. In other words, compared to non-conjugated olefins in which X is a hydrogen atom or an alkyl group, radical addition is more likely to occur when X is $OR^1$.

$R^1$ is a group in which an alkyl group having 1 to 8 carbon atoms is optionally substituted. The number of carbon atoms of the alkyl group having 1 to 8 carbon atoms is, for example, 1, 2, 3, 4, 5, 6, 7, or 8, and may be in the range between the two values exemplified herein. The alkyl group having 1 to 8 carbon atoms may be linear or branched. Examples of the alkyl group include a methyl group, an ethyl group, a propyl group, an isopropyl group, a butyl group, an isobutyl group, a sec-butyl group, a tert-butyl group, a pentyl group, an isopentyl group, a neopentyl group, a 1-ethylpropyl group, a hexyl group, an isohexyl group, a 1,1-dimethylbutyl group, a 2,2-dimethylbutyl group, a 3,3-dimethylbutyl group, a 2-ethylbutyl group, a heptyl group, an octyl group, an isooctyl group, and a 2-ethylhexyl group. The alkyl group is preferably a methyl group, an ethyl group, or a propyl group. $R^1$ may be optionally substituted, and examples of the substituent include a hydroxy group, an isocyanate group, a carboxy group, an amino group, a sulfo group, and an alkoxysilyl group. The group in which an alkyl group having 1 to 8 carbon atoms is optionally substituted can be a group selected from among an unsubstituted alkyl group, $(CH_2)nOH$, $(CH_2)nNCO$, $(CH_2)nCOOH$, $(CH_2)nNH_2$, $(CH_2)nSO_3H$, and $(CH_2)nOSi(OR^4)_3$. Here, n can be a natural number from 1 to 8, and $R^4$ can be an alkyl group having 1 to 8 carbon atoms.

Each of the above-mentioned X has the effect of promoting radical addition to the vinylidene group, but in addition to the effect, preferably X is selected in consideration of their compatibility with the monomer (for example, a radically polymerizable compound) to be used. The compatibility of the monomer and X can be judged from the copolymerizability of the monomer and the substituted ethylene compound described as $CH_2=C(H)X$. When the monomer is $M_1$ and the substituted ethylene compound is $M_2$, the monomer reactivity ratio $r_1$ represented by the Mayo-Lewis formula is preferably 0.1 to 5, more preferably 0.3 to 2, and even more preferably 0.5 to 1.5. For example, when X is an acetoxy group, the above substituted ethylene compound is vinyl acetate. Since the reactivity ratio of styrene and vinyl acetate is $r_1=22$, a chain transfer agent in which X is an acetoxy group hardly functions in a styrene polymerization system and is not preferable. On the other hand, when X is $COOCH_3$, the above substituted ethylene compound is methyl acrylate. Since the reactivity ratio of styrene and methyl acrylate is $r_1=0.75$, a chain transfer agent in which X is a $COOCH_3$ group is suitable for the styrene polymerization system. When a substituted ethylene compound described as $CH_2=C(H)X$ is used as a monomer, the monomer $M_1$ and the substituted ethylene compound $M_2$ to be compared are the same, so $r_1=1$. That is, it is most preferable to select X that is the same as the substituent of the monomer to be used. For example, it is most preferable that the chain transfer agent used in the polymerization system of methyl acrylate is $X=COOCH_3$. The chain transfer agent of an embodiment of the present invention is a chain transfer agent used during polymerization of the monomer $M_1$, and preferably the monomer $M_1$ contains X as a substituent. X may not contain sulfur.

Each Y and Z is a group selected from among a cyano group, an optionally substituted aryl group, an acyl group, a carboxy group, $COOR^1$, $C(O)NH_2$, $C(O)NHR^1$, $C(O)NR^1{}_2$, $SOR^1$, $SO_2R^1$, $SO_3R^1$, $OR^1$, and a halogen, or Y and Z may be bonded to form an optionally substituted carbocyclic structure or an optionally substituted heterocyclic structure. Specific examples of the aryl group and the acyl group include those listed in the description of X.

In an embodiment of the present invention, Y and/or Z are groups that determine the ease of a cleavage reaction caused by a radical added to the vinylidene group. The more effective Y and/or Z are in stabilizing the radical on the carbon atom to which they are bonded, the more likely the cleavage reaction is to occur. Therefore, groups that can be expected to delocalize the unpaired electron due to the resonance effect of Y and/or Z are preferred, and each of Y and/or Z is preferably a group selected among from a cyano group, an optionally substituted aryl group, an acyl group, a carboxy group, $COOR^1$, $C(O)NH_2$, $C(O)NHR^1$, $C(O)NR^1{}_2$, $SOR^1$, $SO_2R^1$, $SO_3R^1$, $OC(O)R^1$, $N(H)C(O)R^1$ and a halogen.

In an embodiment of the present invention, Y and/or Z also determine the ease of a reinitiation reaction by a radical species freed by the cleavage reaction. The reinitiation reaction is likely to occur when the monomer reactivity ratio $r_2$ represented by the Mayo-Lewis formula in the copolymerization is 0 to 5, where $M_2$ is a monomer represented by $CH_2=C(Y)Z$ and $M_1$ is a monomer used in polymerization. The reinitiation reaction is more likely to occur when $r_2$ is 0 to 2, and particularly likely to occur when $r_2$ is 0 to 1. However, even in the case of a so-called destructive chain transfer reaction in which no reinitiation reaction occurs at all, the purpose of adjusting the molecular weight can be achieved without any problems. Therefore, preferably Y and/or Z are selected primarily based on the likelihood of the reinitiation reaction as described in the previous paragraph, and the range of selection is not restricted by the presence or absence of the reinitiation reaction.

$R^1$ which may be contained in Y and/or Z is a group in which an alkyl group having 1 to 8 carbon atoms is optionally substituted. The number of carbon atoms of the alkyl group having 1 to 8 carbon atoms may be, for example, 1, 2, 3, 4, 5, 6, 7, or 8, and may be in the range between the two values exemplified herein. The alkyl group having 1 to 8 carbon atoms may be linear or branched. Examples of the alkyl group include a methyl group, an ethyl group, a propyl group, an isopropyl group, a butyl group, an isobutyl group, a sec-butyl group, a tert-butyl group, a pentyl group, an isopentyl group, a neopentyl group, a 1-ethylpropyl group, a hexyl group, an isohexyl group, a 1,1-dimethylbutyl group, a 2,2-dimethylbutyl group, a 3,3-dimethylbutyl group, a 2-ethylbutyl group, a heptyl group, an octyl group, an isooctyl group, and a 2-ethylhexyl group. The alkyl group is preferably a methyl group, an ethyl group, or a propyl group. $R^1$ may be optionally substituted, and

examples of the substituent include a hydroxy group, an isocyanate group, a carboxy group, an amino group, a sulfo group, and an alkoxysilyl group. The group in which an alkyl group having 1 to 8 carbon atoms is optionally substituted can be a group selected from among an unsubstituted alkyl group, $(CH_2)nOH$, $(CH_2)nNCO$, $(CH_2)nCOOH$, $(CH_2)nNH_2$, $(CH_2)nSO_3H$, and $(CH_2)nOSi(OR^4)_3$. Here, n can be a natural number from 1 to 8, and $R^4$ can be an alkyl group having 1 to 8 carbon atoms.

Y and Z may be bonded to form an optionally substituted carbocyclic structure or an optionally substituted heterocyclic structure.

The carbocyclic structure may be a cycloalkyl group having 3 to 8 carbon atoms.

The heterocyclic structure may be a 3-8 membered heterocyclic ring. The heterocyclic ring has at least one heteroatom selected from among N, O, and S, and may have two or more heteroatoms. Preferably, the heterocyclic ring has at least one O.

The carbocyclic structure and the heterocyclic structure may have a substituent, and examples of the substituent include an alkyl group having 1 to 8 carbon atoms, a cyano group, a carboxy group, an oxo group, and a halogen. Preferably, the substituent includes an oxo group.

In an embodiment of the present invention, Y and Z may be bonded to form Meldrum's acid or a Meldrum's acid derivative.

X, Y, and Z may be different from each other.

Furthermore, with regard to X, Y, and Z, at least two of X, Y, and Z may be the same, Y and Z may be the same, or Y and Z may be the same while X may be different.

X, Y, and Z may all be the same.

Q is hydrogen, an optionally substituted alkyl group having 1 to 6 carbon atoms, or a phenyl group. Q can be an optionally substituted alkyl group having 1 to 6 carbon atoms, and examples of the substituent include a cyano group, a carboxy group, a halogen, a hydroxy group, an alkoxy group, and an amino group. Q is preferably hydrogen.

$R^2$ and $R^3$ are groups selected from among hydrogen, an alkyl group having 2 to 6 carbon atoms, an optionally substituted aryl group, and an optionally substituted heterocyclic structure, except when both $R^2$ and $R^3$ are hydrogen. When both $R^2$ and $R^3$ are hydrogen, a terminal olefin is generated by the chain transfer reaction. On the other hand, when at least one of $R^2$ and $R^3$ is a group selected from among an alkyl group having 2 to 6 carbon atoms, an optionally substituted aryl group, and an optionally substituted heterocyclic structure, an internal olefin is generated by the chain transfer reaction. The internal olefin is more stable than the terminal olefin because of a hyperconjugation effect or a resonance effect from the substituent. For this reason, compared with a chain transfer agent in which $R^2$ and $R^3$ are both hydrogen and which generates a terminal olefin, a chain transfer agent in which at least one of $R^2$ and $R^3$ is a group selected from among an alkyl group having 2 to 6 carbon atoms, an optionally substituted aryl group, and an optionally substituted heterocyclic structure, which generates a more stable internal olefin, is more likely to cause a chain transfer reaction. In addition, the stabilization of the olefin and the increase in steric hindrance near the olefin, both by $R^2$ or $R^3$, thermodynamically and kinetically suppress the re-attack of radical species, respectively. As a result, the effect of making the chain transfer reaction as an irreversible reaction and the effect of suppressing the formation of branched and/or crosslinked structures originating from the generated olefin terminals are achieved. Thus, the effects of $R^2$ and $R^3$ are to promote the chain transfer reaction and to suppress side reactions caused by the olefin terminals generated after the chain transfer.

$R^2$ and/or $R^3$ can be an alkyl group having 2 to 6 carbon atoms. The number of carbon atoms of the alkyl group is, for example, 2, 3, 4, 5, or 6, and may be in the range between the two values exemplified herein. The alkyl group having 2 to 6 carbon atoms may be linear or branched. Examples of the alkyl group include an ethyl group, a propyl group, an isopropyl group, a butyl group, an isobutyl group, a sec-butyl group, a tert-butyl group, a pentyl group, an isopentyl group, a neopentyl group, a 1-ethylpropyl group, a hexyl group, an isohexyl group, a 1,1-dimethylbutyl group, a 2,2-dimethylbutyl group, a 3,3-dimethylbutyl group, and a 2-ethylbutyl group. When $R^2$ and/or $R^3$ are methyl groups, internal olefins are also generated, so that the same effect as that of an alkyl group having 2 to 6 carbon atoms such as an ethyl group can be expected in terms of promoting the chain transfer reaction. However, in terms of suppressing the attack of radicals on the olefin terminal generated after chain transfer, the steric hindrance of the methyl group is insufficient. This can be presumed from the fact that methyl crotonate, an internal olefin in which a methyl group is substituted at the $\beta$-position of methyl acrylate, undergoes radical copolymerization with other vinyl monomers. Therefore, $R^2$ and/or $R^3$ are preferably more bulky alkyl groups having 2 to 6 carbon atoms, and are particularly preferably a sec-butyl group, a tert-butyl group, or an isopropyl group.

$R^2$ and/or $R^3$ can be an optionally substituted aryl group. When the internal olefin is generated by a chain transfer reaction, the aryl group can resonate with the olefin. The formation of a long and stable conjugated system promotes the chain transfer reaction, and also has the effect of suppressing the reverse reaction. The stabilization of the olefin by the resonance effect is significantly more effective than the stabilization by the hyperconjugation effect of the alkyl group, therefore $R^2$ and/or $R^3$ are preferably an aryl group. The aryl group may be a single ring, a condensed ring in which two or more aromatic rings are condensed, or a linked ring in which two or more aromatic rings are linked. The number of carbon

atoms in the aromatic ring constituting the aryl group may be 6 to 18, preferably 6 to 12, and more preferably 6 to 10. Examples of the aryl group include a phenyl group, a 1-naphthyl group, a 2-naphthyl group, a 2-biphenyl group, a 3-biphenyl group, and a 4-biphenyl group, and among these, the aryl group is preferably a phenyl group. The aryl group may have a substituent, and examples of the substituent include an alkyl group having 1 to 8 carbon atoms, an alkoxy group having 1 to 8 carbon atoms, a cyano group, a carboxy group, a halogen, and the like. In particular, a cyano group, a carboxy group, a halogen and the like have the effect of further extending the conjugated system generated by the chain transfer reaction, by resonating with an aromatic ring, thereby particularly preferred as they strongly promote the chain transfer reaction.

$R^2$ and $R^3$ can be an optionally substituted heterocyclic structure.

The heterocyclic structure can be a 3-8 membered heterocyclic ring. The heterocyclic ring has at least one heteroatom selected from among N, O, and S, and can have two or more heteroatoms. The heterocyclic structure can have a substituent, and examples of the substituent include an alkyl group having 1 to 8 carbon atoms, a cyano group, a carboxy group, and a halogen. When the heterocyclic ring is an aliphatic ring, it is possible to expect, as with the alkyl group, the effect of promoting the chain transfer reaction and the effect of suppressing the formation of branched and/or crosslinked structures, derived from the stabilization of internal olefins by hyperconjugation. When the heterocyclic ring is an aromatic ring, it is possible to expect, as with an aryl group, the effect of promoting the chain transfer reactions and the effect of suppressing the formation of branched and/or crosslinked structures based on the stabilization of internal olefins by the resonance effect.

Furthermore, in an embodiment of the present invention, if the steric hindrance of $R^2$ and $R^3$ is too large, radical addition to the vinylidene group is suppressed and the chain transfer constant is reduced. Therefore, from the viewpoint of obtaining an appropriate reactivity, preferably at least one of $R^2$ and $R^3$ is hydrogen.

The chain transfer agent of an embodiment of the present invention is preferably designed by selecting appropriate X, Y, Z, Q, $R^2$, and $R^3$ in general formula (1), based on the structure of the monomer used in the polymerization and whether or not a reinitiation reaction occurs. For example, in a radical polymerization system of acrylic esters, when designing a chain transfer agent including a reinitiation reaction,

X is a group selected from among a cyano group, a carboxy group, $COOR^1$, $C(O)NH_2$, $C(O)NHR^1$, $C(O)NR^1{}_2$, $SOR^1$, $SO_2R^1$, $SO_3R^1$, and a phenyl group,
each Y and Z is a group selected from among a cyano group, a carboxy group, $COOR^1$, $C(O)R^1$, $C(O)NH_2$, $C(O)NHR^1$, $C(O)NR^1{}_2$, $SOR^1$, $SO_2R^1$, $SO_3R^1$, and a phenyl group,
X, Y, and Z are the same or different,
the above $R^1$ is an alkyl group having 1 to 8 carbon atoms,
Q is hydrogen or a methyl group,
one of $R^2$ and $R^3$ can be hydrogen, and the other can be a group selected from among an alkyl group having 2 to 6 carbon atoms, an optionally substituted aryl group, and an optionally substituted heterocyclic structure.

Among these, from the viewpoints of the ease of the chain transfer, compatibility with monomers and polymers, and solubility, it is preferable that,

X is a group selected from among a cyano group, $COOR^1$, and a phenyl group,
each Y and Z is a group selected from among a cyano group, $COOR^1$, and a phenyl group,
X, Y, and Z are the same or different,
the above $R^1$ is an alkyl group having 1 to 8 carbon atoms,
Q is hydrogen or a methyl group,
one of $R^2$ and $R^3$ is hydrogen, and the other is a group selected from among an alkyl group having 2 to 6 carbon atoms, an optionally substituted aryl group, and an optionally substituted heterocyclic structure.

Furthermore, from the viewpoint of synthesis, it is particularly preferred that,

X is a group selected from among a cyano group and $COOR^1$,
each Y and Z is a group selected from among a cyano group, $COOR^1$ and a phenyl group,
X, Y and Z are the same or different,
the above $R^1$ is an alkyl group having 1 to 8 carbon atoms,
Q is a methyl group,
one of $R^2$ and $R^3$ is hydrogen and the other is a phenyl group.

The chain transfer agent of an embodiment of the present invention has a structure represented by following general formula (1) and may have a skeleton represented by general formula (23) described below.

The chain transfer agent having a skeleton represented by following general formula (23) will be described.

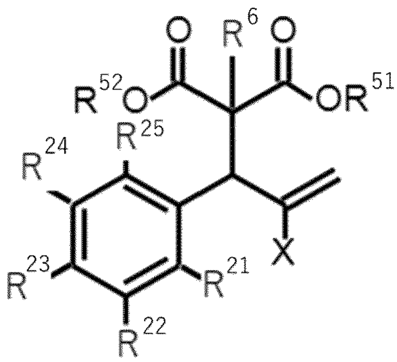

(23)

The reaction mechanism when the compound represented by general formula (23) functions as a chain transfer agent for radical polymerization can be explained as follows. The mechanism is considered that first, an addition reaction occurs in which the vinylidene group accepts radical species, followed by a cleavage reaction, to generate a polymer chain having an end represented by general formula (21) and a radical represented by following general formula (24). The generation of the radical represented by general formula (24) initiates regrowth of the polymer. The series of the above reaction mechanisms is called an addition-cleavage reaction, in which X is related to the ease of the addition reaction, and $R^{51}$, $R^{52}$, and $R^6$, and $R^{21}$, $R^{22}$, $R^{23}$, $R^{24}$, and $R^{25}$ are related to the ease of the cleavage reaction. Here, preferably an optimal X is selected according to the expected monomer. When a reinitiation reaction from radical species freed by the chain transfer reaction is expected, preferably optimal $R^{51}$, $R^{52}$, and $R^6$ are selected according to the expected monomer.

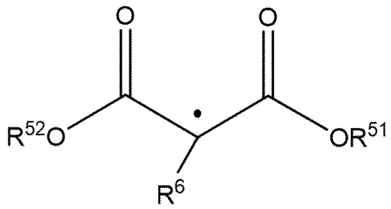

(24)

In general formula (23), X is a group selected from among a cyano group, an optionally substituted aryl group, an acyl group, a carboxy group, $COOR^1$, $C(O)NH_2$, $C(O)NHR^1$, $C(O)NR^1{}_2$, $SOR^1$, $SO_2R^1$, $SO_3R^1$, and $OR^1$.

In one embodiment of the present invention, X can be a cyano group. When X is a cyano group, an induction effect of a nitrogen atom and a resonance effect of the cyano group and the vinylidene group work to create a bias (polarization) of electron density between the two carbon atoms comprising the vinylidene group. As a result, the vinylidene group is more likely to accept the addition of radical species. Furthermore, when a radical is added to the vinylidene group, a new radical species is generated, but the cyano group also has a function of delocalizing the unpaired electron of this radical by the resonance effect and stabilizing the radical species. In other words, compared with non-conjugated olefins in which X is a hydrogen atom or an alkyl group, radical addition is significantly more likely to occur when X is a cyano group.

In one embodiment of the present invention, X can be an optionally substituted aryl group. The aryl group may be a single ring, a condensed ring in which two or more aromatic rings are condensed, or a linked ring in which two or more aromatic rings are linked. The number of carbon atoms in the aromatic ring constituting the aryl group can be 6 to 18, preferably 6 to 12, and more preferably 6 to 10. Examples of the aryl group include a phenyl group, a 1-naphthyl group, a 2-naphthyl group, a 2-biphenyl group, a 3-biphenyl group, and a 4-biphenyl group, and the aryl group is preferably a phenyl group. The aryl group can have a substituent, and examples of the substituent include an alkyl group having 1 to 8 carbon atoms, a cyano group, and a carboxy group. When X is an optionally substituted aryl group, a resonance effect of the aryl group and the vinylidene group works, and a bias (polarization) of the electron density occurs between the two carbon atoms constituting the vinylidene group. As a result, the vinylidene group is more likely to accept the addition of radical species. Furthermore, when a radical is added to the vinylidene group, a new radical species is generated, but the aryl group also has a function of delocalizing the unpaired electron of this radical by the resonance effect, stabilizing the radical species. In addition, when the aryl group is substituted with an alkyl group, a cyano group, or a carboxy group, a large resonance effect and a hyperconjugation effect including these substituents work, and the radical is further stabilized compared to an unsubstituted aryl group. From the above, compared with non-conjugated olefins in which X is a hydrogen atom or an alkyl group, radical addition is significantly more likely to occur when X is an optionally substituted aryl group.

In an embodiment of the present invention, X can be an acyl group ($C(=O)R^1$), a carboxy group, $COOR^1$, an amide group ($C(O)NH_2$, $C(O)NHR^1$, or $C(O)NR^1{}_2$), or a sulfone group-containing substituent $SOR^1$, $SO_2R^1$, or $SO_3R^1$.

Also, in an embodiment of the present invention, X can be an acyl group ($C(=O)R^1$), a carboxy group, $COOR^1$, or an amide group ($C(O)NH_2$, $C(O)NHR^1$, or $C(O)NR^1{}_2$). Examples of the acyl group include a formyl group, an acetyl group, a propionyl group, and a benzoyl group. Examples of $COOR^1$, that is, an alkoxycarbonyl group ($R^1OC(=O)$), include a methoxycarbonyl group, an ethoxycarbonyl group, and a tert-butoxycarbonyl group. Among the amide groups, examples of the N-substituted amide group ($C(O)NHR^1$ or $C(O)N\,R^1{}_2$) include an N-substituted amide group in which $R^1$ is a methyl group, an ethyl group, an n-propyl group, or an isopropyl group. When X is an acyl group ($C(=O)R^1$), a carboxy group, $COOR^1$, an amide group ($C(O)NH_2$, $C(O)NHR^1$, or $C(O)NR^1{}_2$), or a sulfone group-containing substituent $SOR^1$, $SO_2R^1$, or $SO_3R^1$, an inductive effect of the oxygen atom of the carbonyl/sulfone group and a resonance effect of the carbonyl/sulfone group and the vinylidene group work, causing a bias (polarization) of the electron density between the two carbon atoms constituting the vinylidene group. As a result, the vinylidene group becomes more likely to accept the addition of radical species. Furthermore, when a radical is added to the vinylidene group, a new radical species is generated, but the carbonyl/sulfone group also has a function of delocalizing the unpaired electron of this radical by the resonance effect, stabilizing the radical species. In other words, compared with non-conjugated olefins in which X is a hydrogen atom or an alkyl group, radical addition is significantly more likely to occur, when X is an acyl group ($C(=O)R^1$), a carboxy group, $COOR^1$, an amide group ($C(O)NH_2$, $C(O)NHR^1$, or $C(O)NR^1{}_2$), or a sulfone group-containing substituent $SOR^1$, $SO_2R^1$, or $SO_3R^1$.

In an embodiment of the present invention, X can be $OR^1$. When X is $OR^1$, an inductive effect of the oxygen atom and a resonance effect of the oxygen atom and the vinylidene group work, causing a bias (polarization) of the electron density between the two carbon atoms constituting the vinylidene group. As a result, the vinylidene group is more likely to accept the addition of radical species. In other words, compared to non-conjugated olefins in which X is a hydrogen atom or an alkyl group, radical addition is more likely to occur when X is $OR^1$.

$R^1$ is a group in which an alkyl group having 1 to 8 carbon atoms is optionally substituted. The number of carbon atoms of the alkyl group having 1 to 8 carbon atoms is, for example, 1, 2, 3, 4, 5, 6, 7, or 8, and may be in the range between the two values exemplified herein. The alkyl group having 1 to 8 carbon atoms may be linear or branched. Examples of the alkyl group include a methyl group, an ethyl group, a propyl group, an isopropyl group, a butyl group, an isobutyl group, a sec-butyl group, a tert-butyl group, a pentyl group, an isopentyl group, a neopentyl group, a 1-ethylpropyl group, a hexyl group, an isohexyl group, a 1,1-dimethylbutyl group, a 2,2-dimethylbutyl group, a 3,3-dimethylbutyl group, a 2-ethylbutyl group, a heptyl group, an octyl group, an isooctyl group, and a 2-ethylhexyl group. The alkyl group is preferably a methyl group, an ethyl group, or a propyl group. $R^1$ may be optionally substituted, and examples of the substituent include a hydroxy group, an isocyanate group, a carboxy group, an amino group, a sulfo group, and an alkoxysilyl group. The substituent can be one group selected from among a hydroxy group, an isocyanate group, a carboxy group, an amino group, and an alkoxysilyl group. The group in which an alkyl group having 1 to 8 carbon atoms is optionally substituted can be a group selected from among an unsubstituted alkyl group, $(CH_2)nOH$, $(CH_2)nNCO$, $(CH_2)nCOOH$, $(CH_2)nNH_2$, $(CH_2)nSO_3H$, and $(CH_2)nOSi(OR^4)_3$. Here, n can be a natural number from 1 to 8, and $R^4$ can be an alkyl group having 1 to 8 carbon atoms.

Each of the above-mentioned X has the effect of promoting radical addition to the vinylidene group, but in addition to the effect, preferably X is selected in consideration of their compatibility with the monomer (for example, a radically polymerizable compound) to be used. The compatibility of the monomer and X can be judged from the copolymerizability of the monomer and the substituted ethylene compound described as $CH_2=C(H)X$. When the monomer is $M_1$ and the substituted ethylene compound is $M_2$, the monomer reactivity ratio $r_1$ represented by the Mayo-Lewis formula is preferably 0.1 to 5, more preferably 0.3 to 2, and even more preferably 0.5 to 1.5. For example, when X is an acetoxy group, the above substituted ethylene compound is vinyl acetate. Since the reactivity ratio of styrene and vinyl acetate is $r_1=22$, a chain transfer agent in which X is an acetoxy group hardly functions in a styrene polymerization system and is not preferable. On the other hand, when X is $COOCH_3$, the above substituted ethylene compound is methyl acrylate. Since the reactivity ratio of styrene and methyl acrylate is $r_1=0.75$, a chain transfer agent in which X is a $COOCH_3$ group is suitable for the styrene polymerization system. When a substituted ethylene compound described as $CH_2=C(H)X$ is used as a monomer, the monomer $M_1$ and the substituted ethylene compound $M_2$ to be compared are the same, so $r_1=1$. That is, it is most preferable to select X that is the same as the substituent of the monomer to be used. For example, it is most preferable that the chain transfer agent used in the polymerization system of methyl acrylate is $X=COOCH_3$. The chain transfer agent of an embodiment of the present invention is a chain transfer agent used during polymerization of the monomer $M_1$, and preferably the monomer $M_1$ contains X as a substituent. X may not contain sulfur.

In an embodiment of the present invention, $COOR^{51}$ and/or $COOR^{52}$ are groups that determine the ease of a cleavage reaction caused by a radical added to the vinylidene group. The more effective $COOR^{51}$ and/or $COOR^{52}$ are in stabilizing the radical on the carbon atom to which they are bonded, the more likely the cleavage reaction is to occur. Therefore, groups that can be expected to delocalize the unpaired electron due to the resonance effect of $COOR^{51}$ and/or $COOR^{52}$ are preferred.

In an embodiment of the present invention, $COOR^{51}$ and/or $COOR^{52}$ also determine the ease of a reinitiation reaction by a radical species freed by the cleavage reaction. The reinitiation reaction is likely to occur when the monomer

reactivity ratio $r_2$ represented by the Mayo-Lewis formula in the copolymerization is 0 to 5, where $M_2$ is a monomer represented by $CH2=C(COOR^{51})COOR^{52}$ and $M_1$ is a monomer used in polymerization. The reinitiation reaction is more likely to occur when $r_2$ is 0 to 2, and particularly likely to occur when $r_2$ is 0 to 1. However, even in the case of a so-called destructive chain transfer reaction in which no reinitiation reaction occurs at all, the purpose of adjusting the molecular weight can be achieved without any problems. Therefore, preferably $COOR^{51}$ and/or $COOR^{52}$ are selected primarily based on the ease of the reinitiation reaction as described in the previous paragraph, and the range of selection is not restricted by the presence or absence of the reinitiation reaction.

$R^{51}$ and $R^{52}$ are alkyl groups having 1 to 8 carbon atoms, and $R^{51}$ and $R^{52}$ are the same or different. The number of carbon atoms of the alkyl group having 1 to 8 carbon atoms is, for example, 1, 2, 3, 4, 5, 6, 7, or 8, and may be in the range between the two values exemplified herein. The alkyl group having 1 to 8 carbon atoms may be linear or branched. Examples of the alkyl group include a methyl group, an ethyl group, a propyl group, an isopropyl group, a butyl group, an isobutyl group, a sec-butyl group, a tert-butyl group, a pentyl group, an isopentyl group, a neopentyl group, a 1-ethylpropyl group, a hexyl group, an isohexyl group, a 1,1-dimethylbutyl group, a 2,2-dimethylbutyl group, a 3,3-dimethylbutyl group, a 2-ethylbutyl group, a heptyl group, an octyl group, an isooctyl group, and a 2-ethylhexyl group.

$R^6$ is hydrogen, an optionally substituted alkyl group having 1 to 6 carbons, or a phenyl group. $R^6$ may be an optionally substituted alkyl group having 1 to 6 carbons, and examples of the substituent include a cyano group, a carboxy group, a halogen, a hydroxy group, an alkoxy group, and an amino group. $R^6$ is preferably a methyl group.

In general formula (23), $R^{21}$, $R^{22}$, $R^{23}$, $R^{24}$, and $R^{25}$ are selected from among hydrogen, an alkyl group having 1 to 8 carbon atoms, an alkoxy group having 1 to 8 carbon atoms, a cyano group, a carboxy group, and a halogen. $R^{21}$, $R^{22}$, $R^{23}$, $R^{24}$, and $R^{25}$ are the same or different. The number of carbon atoms in the alkyl group having 1 to 8 carbon atoms is, for example, 1, 2, 3, 4, 5, 6, 7, or 8, and may be in the range between the two values exemplified herein. The alkyl group having 1 to 8 carbon atoms may be linear or branched. Examples of the alkyl group include a methyl group, an ethyl group, a propyl group, an isopropyl group, a butyl group, an isobutyl group, a sec-butyl group, a tert-butyl group, a pentyl group, an isopentyl group, a neopentyl group, a 1-ethylpropyl group, a hexyl group, an isohexyl group, a 1,1-dimethylbutyl group, a 2,2-dimethylbutyl group, a 3,3-dimethylbutyl group, a 2-ethylbutyl group, a heptyl group, an octyl group, an isooctyl group, and a 2-ethylhexyl group. Examples of the alkoxy group include a methoxy group, an ethoxy group, a propoxy group, and a butoxy group. Examples of the halogen include a fluorine group, a chlorine group, a bromine group, and an iodine group. Preferably, $R^{21}$, $R^{22}$, $R^{23}$, $R^{24}$, and $R^{25}$ are hydrogen.

In an embodiment of the present invention, the chain transfer agent having the skeleton represented by the above general formula (23) may not contain sulfur.

The chain transfer agent of the present invention can be used when polymerizing a radically polymerizable compound, and can adjust the weight average molecular weight of the obtained polymer to, for example, 5,000 to 2,000,000. The weight average molecular weight of the polymer is, for example, 5,000, 10,000, 30,000, 50,000, 100,000, 200,000, 500,000, 1,000,000, 1,500,000, or 2,000,000, and may be in the range between the two values exemplified herein.

2. Method for producing chain transfer agent

The method for producing the chain transfer agent of the present invention is not particularly limited, as long as a compound having a structure represented by the above general formula (1) can be obtained.

As an example, the chain transfer agent of the present invention can be obtained by reacting a compound represented by following general formula (2) with a halogen compound represented by general formula (3) (an example shows a bromine compound) in the presence of a solvent and a base.

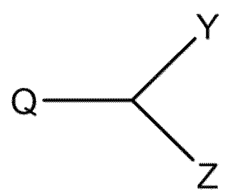

(2)

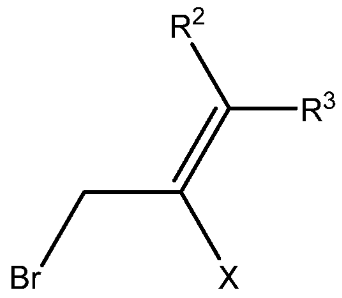

(3)

In the above general formulae (2) and (3), X, Y, Z, Q, $R^2$, and $R^3$ can be the same as X, Y, Z, Q, $R^2$, and $R^3$ in the above-mentioned general formula (1), respectively.

Examples of the solvent to be used include tetrahydrofuran (THF), dichloromethane, chloroform, acetone, 2-butanone, acetonitrile, toluene, ethyl acetate, butyl acetate, methanol, ethanol and the like.

Examples of the base to be used include 1,4-diazabicyclo[2.2.2]octane (DABCO), triethylamine, 1,8-diazabicyclo[5.4.0]undecene-7 (DBU), 1,5-diazabicyclo[4.3.0]nonene-5 (DBN), pyridine and the like.

3. Method for producing polymer using chain transfer agent

3.1 Polymerization step

The method for producing the polymer of the present invention includes a polymerization step of polymerizing a radically polymerizable compound in the presence of the chain transfer agent described above.

According to the production method of the present invention, a polymer with a controlled molecular weight can be obtained.

In the polymerization step of an embodiment of the present invention, one or more chain transfer agents having a skeleton represented by the above general formula (23) may be used. In the polymerization step of an embodiment of the present invention, a chain transfer agent having a skeleton represented by the above general formula (23) may be used in combination with a chain transfer agent other than the chain transfer agent having a skeleton represented by the above general formula (23).

Other chain transfer agents include 2-mercaptoethanol, thioglycerol, 3-mercaptohexan-1-ol, thioglycolic acid, 2-mercaptopropionic acid, 3-mercaptopropionic acid, 4-mercaptobutanoic acid, 6-mercaptohexanoic acid, 11-mercaptoundecanoic acid, 3-mercaptopyruvic acid, 2-mercaptobenzoic acid, 3-mercaptobenzoic acid, 4-mercaptobenzoic acid, thiomalic acid, n-dodecyl mercaptan, pentaerythritol tetrakis(3-mercaptopropionate), $\alpha$-methylstyrene dimer, and naphthoquinone-based compounds.

In the polymerization step of an embodiment of the present invention, when the chain transfer agent used in the polymerization step is 100% by mass, the chain transfer agent having the skeleton represented by the above general formula (23) may be contained in an amount of 50% by mass or more, for example, 50, 55, 60, 65, 70, 75, 80, 85, 90, 95, or 100% by mass, and may be in the range between the two values exemplified herein.

In the polymerization step of an embodiment of the present invention, when the chain transfer agent used in the polymerization step is 100% by mass, the content ratio of the thiol-based chain transfer agent can be, for example, 0, 5, 10, 15, 20, 25, 30, 35, 40, 45, or 50% by mass, and may be in the range between the two values exemplified herein. The thiol-based chain transfer agent can refer to a chain transfer agent containing sulfur as a thiol group, such as a mercaptan compound or the like, and may not include, for example, a chain transfer agent containing sulfur as a sulfone group. In addition, the chain transfer agent having the structure of general formula (23) of the present invention may not be included in the thiol-based chain transfer agent. The examples of the thiol-based chain transfer agent may include 2-mercaptoethanol, thioglycerol, 3-mercaptohexan-1-ol, thioglycolic acid, 2-mercaptopropionic acid, 3-mercaptopropionic acid, 4-mercaptobutanoic acid, 6-mercaptohexanoic acid, 11-mercaptoundecanoic acid, 3-mercaptopyruvic acid, 2-mercaptobenzoic acid, 3-mercaptobenzoic acid, 4-mercaptobenzoic acid, thiomalic acid, and n-dodecyl mercaptan. In the polymerization step of an embodiment of the present invention, the thiol-based chain transfer agent may not be used. In the polymerization step of an embodiment of the present invention, the chain transfer agent containing sulfur may not be used. In addition, in the polymerization step of an embodiment of the present invention, the chain transfer agent may include only a chain transfer agent having a skeleton represented by the above general formula (23).

In the polymerization step of an embodiment of the present invention, the amount of the chain transfer agent used may be appropriately adjusted depending on the type and the like of the radically polymerizable compound used and other polymerization conditions. Although not particularly limited, the amount may be 0.001 to 10 parts by mass with respect to 100 parts by mass of the radically polymerizable compound. The amount of the chain transfer agent used may be, for example, 0.001 parts by mass, 0.002 parts by mass, 0.005 parts by mass, 0.01 parts by mass, 0.02 parts by mass, 0.05

parts by mass, 0.1 parts by mass, 0.2 parts by mass, 0.5 parts by mass, 1.0 parts by mass, 5.0 parts by mass, or 10.0 parts by mass, with respect to 100 parts by mass of the radically polymerizable compound, and may be in the range between the two values exemplified herein.

In the polymerization step of an embodiment of the present invention, the amount of the chain transfer agent used may be 0.01 mmol to 200 mmol with respect to 1 mol of the radically polymerizable compound. The amount of the chain transfer agent used may be, for example, 0.01, 0.02, 0.05, 0.1, 0.2, 0.5, 1, 2, 5, 10, 20, 50, 100, 150, or 200 mmol with respect to 1 mol of the radically polymerizable compound, and may be in the range between the two values exemplified herein.

<Radically polymerizable compound>

The radically polymerizable compound may include a compound having an ethylenically unsaturated bond. Examples of the compound having an ethylenically unsaturated bond include a (meth)acrylic acid alkyl ester, a carboxyl group-containing monomer, a hydroxyl group-containing monomer, an amino group-containing monomer, an amide group-containing monomer, a styrene-based monomer, a cyano group-containing monomer, a monomer having two or more ethylenically unsaturated groups in one molecule, and a derivative thereof.

Examples of the (meth)acrylic acid alkyl ester include methyl (meth)acrylate, ethyl (meth)acrylate, n-propyl (meth)acrylate, isopropyl (meth)acrylate, n-butyl (meth)acrylate, isobutyl (meth)acrylate, t-butyl (meth)acrylate, pentyl (meth)acrylate, hexyl (meth)acrylate, n-heptyl (meth)acrylate, 2-ethylhexyl (meth)acrylate, n-octyl (meth)acrylate, isooctyl (meth)acrylate, nonyl (meth)acrylate, isononyl (meth)acrylate, decyl (meth)acrylate, isodecyl (meth)acrylate, undeca(meth)acrylate, lauryl (meth)acrylate, stearyl (meth)acrylate, and isostearyl (meth)acrylate.

Examples of the carboxy group-containing monomer include acrylic acid, methacrylic acid, β-carboxyethyl (meth)acrylate, 5-carboxypentyl (meth)acrylate, succinic acid mono(meth)acryloyloxyethyl ester, ω-carboxypolycaprolactone mono(meth)acrylate, crotonic acid, maleic acid, fumaric acid, itaconic acid, and citraconic acid.

Examples of the hydroxyl group-containing monomer include 2-hydroxyethyl (meth)acrylate, 2-hydroxypropyl (meth)acrylate, 4-hydroxybutyl (meth)acrylate, 6-hydroxyhexyl (meth)acrylate, 8-hydroxyoctyl (meth)acrylate, 10-hydroxydecyl (meth)acrylate, 12-hydroxylauryl (meth)acrylate, (4-hydroxymethylcyclohexyl)methyl acrylate, N-methylol (meth)acrylamide, vinyl alcohol, allyl alcohol, 2-hydroxyethyl vinyl ether, and 4-hydroxybutyl vinyl ether.

Examples of the amino group-containing monomer include N,N-dialkylaminoalkyl(meth)acrylates such as N,N-dimethylaminoethyl(meth)acrylate and N,N-diethylaminoethyl(meth)acrylate.

Examples of the amide group-containing monomer include (meth)acrylamide, N-alkyl(meth)acrylamides such as N-methyl(meth)acrylamide, N-ethyl(meth)acrylamide, N-propyl(meth)acrylamide, and N-hexyl(meth)acrylamide, N,N-dimethyl(meth)acrylamide, N,N-diethyl(meth)acrylamide, N-vinylpyrrolidone, N-vinylcaprolactam, and (meth)acryloylmorpholine.

Examples of the styrene-based monomer include styrene; alkyl styrenes such as methylstyrene, dimethylstyrene, trimethylstyrene, ethylstyrene, propylstyrene, butylstyrene, hexylstyrene, heptylstyrene, and octylstyrene; halogenated styrenes such as fluorostyrene, chlorostyrene, bromostyrene, and dibromostyrene; and functionalized styrenes such as nitrostyrene, acetylstyrene, and methoxystyrene.

Examples of the cyano group-containing monomer include cyano(meth)acrylate and (meth)acrylonitrile.

Examples of the monomer having two or more ethylenically unsaturated groups in one molecule include ethylene glycol di(meth)acrylate, polyethylene glycol di(meth)acrylate, 1,3-butylene glycol di(meth)acrylate, 1,4-butanediol di(meth)acrylate, 1,6-hexanediol di(meth)acrylate, 1,9-nonanediol di(meth)acrylate, diethylene glycol di(meth)acrylate, triethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, tetraethylene glycol di(meth)acrylate, Examples of the acrylates include cyclodecane dimethanol di(meth)acrylate, trimethylolpropane tri(meth)acrylate, trimethylolpropane ethoxy tri(meth)acrylate, pentaerythritol tri(meth)acrylate, ditrimethylolpropane tetra(meth)acrylate, pentaerythritol tetra(meth) acrylate, pentaerythritol ethoxy tetra(meth)acrylate, dipentaerythritol penta(meth)acrylate, dipentaerythritol hexa(meth) acrylate, and divinylbenzene.

The radically polymerizable compound may be used alone or in combination with two or more kinds. The type and amount of the radically polymerizable compound may be appropriately selected depending on the physical properties of the desired polymer.

<Polymerization initiator>

In the polymerization step, a polymerization initiator may be used. Examples of the polymerization initiator

include a thermal polymerization initiator and a photopolymerization initiator. Examples of the thermal polymerization initiator include an organic peroxide and an azo compound. Examples of the photopolymerization initiator include an aromatic ketone compound and a benzoin compound.

In the polymerization step of an embodiment of the present invention, an azo-based initiator can be used, such as 2,2'-azobis(isobutyronitrile) (AIBN), 2,2'-azobis(2-methylbutyronitrile) (AMBN), 2,2'-azobis(2,4-dimethylvaleronitrile) (ADVN), 1,1'-azobis(1-cyclohexanecarbonitrile) (ACHN), dimethyl-2,2'-azobisisobutyrate (MAIB), and 4,4'-azobis(4-cyanovaleric acid) (ACVA).

Examples of the peroxide-based polymerization initiator include t-butyl hydroperoxide, cumene hydroxide, dicumyl peroxide, benzoyl peroxide, lauroyl peroxide, caproyl peroxide, di-i-propyl peroxydicarbonate, di-2-ethylhexyl peroxydicarbonate, t-butyl peroxypivalate, 2,2-bis(4,4-di-t-butylperoxycyclohexyl)propane, 2,2-bis(4,4-di-t-amylperoxycyclohexyl)propane, 2,2-bis(4,4-di-t-octylperoxycyclohexyl)propane, 2,2-bis(4,4-di-α-cumylperoxycyclohexyl)propane, 2,2-bis(4,4-di-t-butylperoxycyclohexyl)butane, 2,2-bis(4,4-di-t-octylperoxycyclohexyl)butane, ammonium persulfate, potassium persulfate, and sodium persulfate.

In the polymerization step of an embodiment of the present invention, the polymerization initiator may be used alone or in combination with other polymerization initiator. The polymerization initiator may be used in an amount of 0.001 to 5 parts by mass with respect to 100 parts by mass of the radically polymerizable compound.

<Solvent>

In the polymerization step of an embodiment of the present invention, known solvents can be used.

As an example, the solvents used in the polymerization include aromatic hydrocarbons such as benzene, toluene, and xylene; aliphatic hydrocarbons such as n-pentane, n-hexane, n-heptane, and n-octane; alicyclic hydrocarbons such as cyclopentane, cyclohexane, cycloheptane, and cyclooctane; ethers such as diethyl ether, diisopropyl ether, 1,2-dimethoxyethane, dibutyl ether, tetrahydrofuran, dioxane, anisole, phenylethyl ether, and diphenyl ether; halogenated hydrocarbons such as chloroform, carbon tetrachloride, 1,2-dichloroethane, and chlorobenzene; esters such as ethyl acetate, propyl acetate, butyl acetate, and methyl propionate; ketones such as acetone, methyl ethyl ketone, diethyl ketone, methyl isobutyl ketone, and cyclohexanone; amides such as N,N-dimethylformamide, N,N-dimethylacetamide, and N-methylpyrrolidone; nitriles such as acetonitrile and benzonitrile; and sulfoxides such as dimethyl sulfoxide and sulfolane.

In the polymerization step of an embodiment of the present invention, the radically polymerizable compound can be polymerized in one or more organic solvents selected from among ethyl acetate, ethyl methyl ketone, and toluene. As an example, the adhesive composition of an embodiment of the present invention can be obtained by a production method comprising a polymerization step of polymerizing the radically polymerizable compound in one or more organic solvents selected from among ethyl acetate, ethyl methyl ketone, and toluene.

As another example, in the polymerization step of an embodiment of the present invention, the radically polymerizable compound can be polymerized in an aqueous medium. The aqueous medium means water or a medium mainly composed of water. Specifically, examples of the aqueous medium include water alone, a mixture of water and a lower alcohol such as methanol or ethanol, but among these, water alone is preferred. As an example, the polymer particles of an embodiment of the present invention can be obtained by a production method comprising a polymerization step of polymerizing the radically polymerizable compound in an aqueous medium.

The weight average molecular weight of the polymer obtained by the above-mentioned production method can be, for example, 5,000 to 2,000,000. The weight average molecular weight of the polymer can be, for example, 5,000, 10,000, 30,000, 50,000, 100,000, 200,000, 500,000, 1,000,000, 1,500,000, or 2,000,000, and may be in the range between the two values exemplified herein.

3.2 Other compound blending step

The production method of an embodiment of the present invention can include a compound blending step of adding a necessary compound to the obtained polymer according to the purpose. As an example, the production method of an embodiment of the present invention can comprise a polymerization step and a crosslinking agent blending step, and in the crosslinking agent blending step, at least one crosslinking agent selected from among an isocyanate crosslinking agent, an epoxy crosslinking agent, and a metal chelate crosslinking agent can be blended into the obtained polymer. As described below, particularly when the polymer is used as an adhesive composition, a crosslinking agent blending step can be included.

Examples of the crosslinking agent include an isocyanate crosslinking agent, an epoxy crosslinking agent, and a metal chelate crosslinking agent. As the isocyanate crosslinking agent, an isocyanate crosslinking agent having two or more isocyanate groups in one molecule can be used. By crosslinking the polymer with the isocyanate crosslinking agent, a crosslinked body can be formed.

The number of isocyanate groups in the isocyanate crosslinking agent can be 2 or more, preferably 2 to 8, and more preferably 3 to 6. When the number of isocyanate groups is within the above range, it is preferable in terms of the efficiency of the crosslinking reaction between the polymer and the isocyanate crosslinking agent, and in terms of maintaining the flexibility of the adhesive layer.

Among isocyanate crosslinking agents, in terms of a good adhesive performance balance and high durability, reaction products of trimethylolpropane and tolylene diisocyanate (L-45, manufactured by Soken Chemical Industries, Ltd., etc.), reaction products of trimethylolpropane and xylylene diisocyanate (TD-75, manufactured by Soken Chemical Industries, Ltd., etc.), and isocyanurates of hexamethylene diisocyanate or tolylene diisocyanate (TSE-100, manufactured by Asahi Kasei, Coronate 2050, manufactured by Tosoh Corporation, etc.) can be used. The isocyanate crosslinking agent may be used alone or in combination of two or more kinds.

As the epoxy crosslinking agent, for example, an epoxy compound having two or more epoxy groups in one molecule can be used. Examples of the epoxy crosslinking agent include ethylene glycol diglycidyl ether, polyethylene glycol diglycidyl ether, glycerin diglycidyl ether, glycerin triglycidyl ether, 1,3-bis(N,N-diglycidylaminomethyl)cyclohexane, N,N,N',N'-tetraglycidyl-m-xylylenediamine, N,N,N',N'-tetraglycidylaminophenylmethane, triglycidyl isocyanurate, m-N,N-diglycidylaminophenyl glycidyl ether, N,N-diglycidyl toluidine, and N,N-diglycidylaniline. The epoxy crosslinking agent may be used alone or in combination of two or more kinds.

Examples of the metal chelate crosslinking agent include compounds in which an alkoxide, acetylacetone, ethyl acetoacetate, or the like is coordinated with a polyvalent metal such as aluminum, iron, copper, zinc, tin, titanium, nickel, antimony, magnesium, vanadium, chromium, zirconium, or the like.

Among these, aluminum chelate compounds are particularly preferred. Specifically, examples include aluminum isopropylate, aluminum secondary butylate, aluminum ethyl acetoacetate diisopropylate, aluminum trisethyl acetoacetate, and aluminum trisacetylacetonate. The metal chelate crosslinking agent may be used alone or in combination of two or more kinds.

In the crosslinking agent blending step, with respect to 100 parts by mass of the polymer, 0.01 to 5 parts by mass of the crosslinking agent can be blended, preferably 0.03 to 4.5 parts by mass, and more preferably 0.05 to 3 parts by mass.

A polymer obtained by the polymer production method of the present invention and a composition containing the crosslinking agent can be made into an adhesive composition.

4. Polymer

The polymer of an embodiment of the present invention has a polymer chain derived from a chain transfer agent represented by the above general formula (1).

The polymer of an embodiment of the present invention contains a polymer chain having a group represented by following general formula (18) at an end thereof.

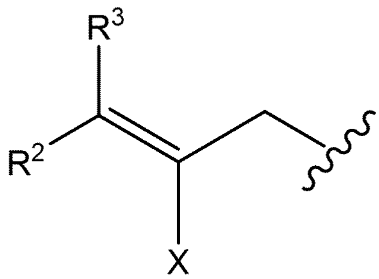

(18)

In general formula (18), X is a group selected from among a cyano group, an optionally substituted aryl group, an acyl group, a carboxy group, $COOR^1$, $C(O)NH_2$, $C(O)NHR^1$,$C(O)NR^1_2$, $SOR^1$, $SO_2R^1$, $SO_3R^1$, and $OR^1$; $R^1$ is an optionally substituted alkyl group having 1 to 8 carbon atoms;

$R^2$ and $R^3$ are groups selected from among hydrogen, an alkyl group having 2 to 6 carbon atoms, an optionally substituted aryl group, and an optionally substituted heterocyclic structure; $R^2$ and $R^3$ are the same or different, and general formula (18) excludes a case where both $R^2$ and $R^3$ are hydrogen. The embodiments of X, $R^1$, $R^2$ and $R^3$ are as described above.

**In** the polymer of an embodiment of the present invention, the group represented by general formula (18) can be represented by following general formula (21).

**In** other words, the polymer of an embodiment of the present invention contains a polymer chain having a group represented by following general formula (21) at an end thereof.

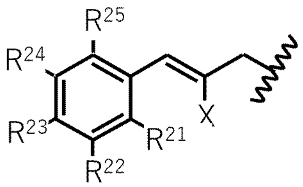

(21)

In general formula (21), $R^{21}$, $R^{22}$, $R^{23}$, $R^{24}$, and $R^{25}$ are groups selected from among hydrogen, an alkyl group having 1 to 8 carbon atoms, an alkoxy group having 1 to 8 carbon atoms, a cyano group, a carboxy group, and a halogen; $R^{21}$, $R^{22}$, $R^{23}$, $R^{24}$, and $R^{25}$ are the same or different;

X is a group selected from among a cyano group, an optionally substituted aryl group, an acyl group, a carboxy group, $COOR^1$, $C(O)NH_2$, $C(O)NHR^1$, $C(O)NR^1_2$, $SOR^1$, $SO_2R^1$, $SO_3R^1$, and $OR^1$; $R^1$ is an optionally substituted alkyl group having 1 to 8 carbon atoms.

The embodiments of $R^{21}$, $R^{22}$, $R^{23}$, $R^{24}$, and $R^{25}$, X, $R^1$ are as described above.

In the polymer of an embodiment of the present invention, a polymer chain having a group represented by general formula (18) at an end thereof may further have a group represented by following general formula (19) at an end thereof.

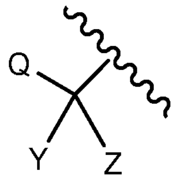

(19)

Each Y and Z is a group selected from among a cyano group, an optionally substituted aryl group, an acyl group, a carboxy group, $COOR^1$, $C(O)NH_2$, $C(O)NHR^1$, $C(O)NR^1_2$, $SOR^1$, $SO_2R^1$, $SO_3R^1$, $OR^1$, $OC(O)R^1$, $N(H)C(O)R^1$, and a halogen, or Y and Z are bonded to form an optionally substituted carbocyclic structure or an optionally substituted heterocyclic structure.

Y and Z are the same or different; $R^1$ is an optionally substituted alkyl group having 1 to 8 carbon atoms.

Q is hydrogen, an optionally substituted alkyl group having 1 to 6 carbon atoms, or a phenyl group.

In the polymer of an embodiment of the present invention, the group represented by general formula (19) can be represented by general formula (22).

In other words, in the polymer of an embodiment of the present invention, a polymer chain having a group represented by following general formula (21) at an end thereof can further have a group represented by following general formula (22) at an end thereof.

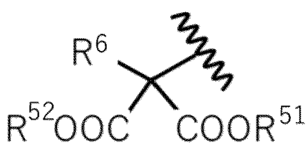

(22)

In general formula (22), $R^{51}$ and $R^{52}$ are alkyl groups having 1 to 8 carbon atoms; $R^{51}$ and $R^{52}$ are the same or different;

$R^6$ is hydrogen, an optionally substituted alkyl group having 1 to 6 carbon atoms, or a phenyl group.

The embodiments of $R^{51}$, $R^{52}$, $R^6$ are as described above.

The polymer having the above-mentioned terminal structure can be obtained by polymerizing a radically polymerizable compound in the presence of a chain transfer agent having a specific skeleton, specifically, general formula (1), particularly general formula (23). As described above, the reaction mechanism when the compound having general formula (1), particularly having general formula (23), functions as a chain transfer agent for radical polymerization is considered to be that an addition reaction in which the vinylidene group accepts a radical species occurs first, followed by a cleavage reaction to generate radicals.

The following describes an example in which a compound having general formula (23) is used. **In** this process, a polymer chain having a terminal structure represented by general formula (21) may be generated first. The polymer chain may have a terminal structure represented by general formula (21) at one end, and a terminal structure represented by general formula (22) or a terminal structure derived from an initiator at the other end.

Therefore, the polymer of an embodiment of the present invention may contain the polymer chain having a group represented by following general formula (21) at the end thereof. Furthermore, the polymer of an embodiment of the present invention may include:

- a polymer chain having a group represented by general formula (21) at one end and a group represented by general formula (22) at the other end, and/or
- a polymer chain having a group represented by general formula (21) at one end and a terminal structure derived from an initiator at the other end.

Note that the polymer of an embodiment of the present invention may have a polymer chain other than the polymer chain having the above structure.

The polymer of an embodiment of the present invention may have a content ratio of sulfur derived from the thiol-based chain transfer agent of 100 ppm or less. The content ratio of sulfur derived from the thiol-based chain transfer agent in the polymer of an embodiment of the present invention is, for example, 0, 10, 20, 30, 40, 50, 60, 70, 80, 90, or 100 ppm, and may be in the range between the two values exemplified herein. The thiol-based chain transfer agent is as described above. The polymer of an embodiment of the present invention may not contain a -C-S-C- structure, which may generally be contained in a polymer obtained when polymerization is performed using a thiol-based chain transfer agent.

The polymer of an embodiment of the present invention may have a content ratio of total sulfur of 100 ppm or less, including sulfur derived from the chain transfer agent having the structure of general formula (23) of the present invention, and from the radically polymerizable compound, and the like. The content ratio of sulfur in the polymer of an embodiment of the present invention is, for example, 0, 10, 20, 30, 40, 50, 60, 70, 80, 90, or 100 ppm, and may be in the range between the two values exemplified herein.

The polymer of an embodiment of the present invention has a low content ratio of sulfur derived from a thiol-based chain transfer agent which is used in the polymerization of conventional polymers, and therefore is less likely to cause problems that are considered to be caused by sulfur, such as corrosion problems.

The polymer of an embodiment of the present invention may contain a repeating monomer unit, and may contain a repeating monomer unit derived from a radically polymerizable compound. The radically polymerizable compound may contain the radically polymerizable compound described above, and may contain a compound having an ethylenically unsaturated bond. Examples of the compound having an ethylenically unsaturated bond include (meth)acrylic acid alkyl ester, a carboxyl group-containing monomer, a hydroxyl group-containing monomer, an amino group-containing monomer, an amide group-containing monomer, a styrene-based monomer, a cyano group-containing monomer, a monomer having two or more ethylenically unsaturated groups in one molecule, and a derivative thereof.

In the polymer of an embodiment of the present invention, the repeating monomer unit may have at least one group selected from among a carboxy group, a hydroxyl group, an amino group, and an amide group. Here, the carboxy group, the hydroxyl group, the amino group, and the amide group may be derived from a radically polymerizable compound. In other words, the polymer of an embodiment of the present invention may have a repeating monomer unit derived from at least one monomer selected from among a carboxy group-containing monomer, a hydroxyl group-containing monomer, an amino group-containing monomer, and an amide group-containing monomer. The embodiment in which the repeating monomer unit has at least one group selected from among a carboxy group, a hydroxyl group, an amino group, and an amide group is particularly suitable when the polymer is used as an adhesive composition, as described below.

The weight average molecular weight (Mw) of the polymer of an embodiment of the present invention can be, for example, 1000 to 2,000,000. The weight average molecular weight of the polymer can be, for example, 1000, 2000, 3000, 5000, 10,000, 20,000, 30,000, 50,000, 100,000, 200,000, 300,000, 500,000, 1,000,000, 1,100,000, 1,200,000, 1,300,000, 1,400,000, 1,500,000, 1,600,000, 1,700,000, 1,800,000, 1,900,000, or 2,000,000, and may be in the range between the two values exemplified herein.

The molecular weight distribution (weight average molecular weight Mw/number average molecular weight Mn) of the polymer of an embodiment of the present invention can be, for example, 1.1 to 4.5. The Mw/Mn of the polymer can be, for example, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, or 4.5, and may be in the range between the two values exemplified herein.

The polymer of the present invention is unlikely to cause a corrosion problem, thereby utilizing such characteristics, it can be used as a material for various applications. As an example, the polymer of an embodiment of the present invention can be used for an adhesive composition and polymer particles.

5. Adhesive Composition

The adhesive composition of an embodiment of the present invention contains the above-mentioned polymer.

The adhesive composition of an embodiment of the present invention preferably contains the above-mentioned polymer and at least one crosslinking agent selected from among an isocyanate crosslinking agent, an epoxy crosslinking agent, and a metal chelate crosslinking agent. Examples of the isocyanate crosslinking agent, the epoxy crosslinking agent, and the metal chelate crosslinking agent include the above-mentioned isocyanate crosslinking agent, epoxy crosslinking agent, and metal chelate crosslinking agent.

The adhesive composition of an embodiment of the present invention contains a polymer having repeating monomer units having at least one group selected from among a carboxy group, a hydroxyl group, an amino group, and an amide group, and also contains the above-mentioned crosslinking agent to form a cross-linked body, thereby improving the adhesive properties.

The adhesive composition of an embodiment of the present invention may contain 0.01 to 5 parts by mass of the crosslinking agent with respect to 100 parts by mass of the polymer. The content of the crosslinking agent is, for example, 0.01, 0.05, 0.1, 0.2, 0.3, 0.5, 1.0, 1.5, 2.0, 2.5, 3.0, 3.5, 4.0, 4.5, or 5.0 parts by mass, and may be in the range between the two values exemplified herein.

The adhesive composition of an embodiment of the present invention may contain, in addition to the crosslinking agent, a known compound that can be added to the adhesive composition, such as a tackifier resin and the like.

The method for production of the adhesive composition of an embodiment of the present invention is not particularly limited, but preferably includes a crosslinking agent blending step of blending at least one crosslinking agent selected from among an isocyanate crosslinking agent, an epoxy crosslinking agent, and a metal chelate crosslinking agent, with the polymer of the present invention. The polymer of the present invention preferably includes a polymerization step of polymerizing a radically polymerizable compound in the presence of a chain transfer agent having a skeleton represented by general formula (23) and a polymerization initiator, and the radically polymerizable compound preferably includes at least one monomer selected from among a carboxyl group-containing monomer, a hydroxyl group-containing monomer, an amino group-containing monomer, and an amide group-containing monomer. The polymerization step is preferably a step of polymerizing the radically polymerizable compound in one or more organic solvents selected from among ethyl acetate, ethyl methyl ketone, and toluene. The polymerization step may be performed under static conditions or under stirring, but is preferably performed under stirring. The polymerization temperature is preferably in the range of 30 to 100°C, and more preferably in the range of 40 to 90°C, depending on the type and amount of the monomer component, the type and amount of the chain transfer agent and the polymerization initiator, and the like. The polymerization temperature may be constant from the start to the end of the polymerization, or may vary within the above range. The polymerization temperature may be controlled by reaction heat alone, or by external heating and cooling. The polymerization time is not particularly limited, but is preferably 1 to 30 hours, and more preferably 2 to 15 hours. In addition, during the polymerization process, monomer components, chain transfer agents, polymerization initiators, and solvents may be appropriately added.

The adhesive sheet of an embodiment of the present invention may contain an adhesive layer formed by the adhesive composition and a substrate. Examples of the adhesive sheet include a double-sided adhesive sheet having only the above-mentioned adhesive layer, a double-sided adhesive sheet having a substrate and the above-mentioned adhesive layer formed on both sides of the substrate, a single-sided adhesive sheet having a substrate and the adhesive layer formed on one side of the substrate, and an adhesive sheet having a release sheet attached to the surface of the adhesive layer of the adhesive sheet that is not in contact with the substrate.

Examples of the substrate and the release sheet include plastic films such as polycarbonate (PC), polymethyl methacrylate (PMMA), polyethylene terephthalate (PET), polybutylene terephthalate (PBT), polyethylene naphthalate (PEN), polyethylene (PE), polypropylene (PP), ethylene-propylene copolymer, ethylene-vinyl acetate copolymer, acrylonitrile-butadiene-styrene copolymer (ABS), polyamide (nylon), polyimide, polyvinyl chloride (PVC), and the like. Examples of the substrate may also include glass, paper, nonwoven fabric, and the like.

The adhesive layer can be obtained by applying the above-mentioned adhesive composition onto the substrate or a release-treated surface of the release sheet, and removing the solvent by heating. Heating conditions can be adjusted according to the type of the solvent. The heating temperature can be, for example, 50 to 150°C. The heating time can be, for example, 1 to 10 minutes. The thickness of the dried coating film can be, for example, 5 to 100 μm.

After the release sheet is attached onto the coating film formed under the above conditions, the adhesive layer can be aged for 3 days or more in an environment of 5 to 60°C and 30 to 70% RH.

The adhesive composition can be applied and dried to a predetermined thickness using a known method, such as spin coating, knife coating, roll coating, bar coating, blade coating, die coating, or gravure coating.

## 6. Polymer Particles

The polymer particles of an embodiment of the present invention contain the above-mentioned polymer. The polymer particles preferably have an average particle size of 0.05 to 500 μm. The average particle size may be, for example, 0.05, 0.10, 0.20, 0.50, 1, 2, 5, 10, 20, 50, 100, 150, 200, 250, 300, 350, 400, 450, or 500 μm, and may be in the range between the two values exemplified herein. The average particle size can be the average particle size measured by the method described in the Examples.

The polymer particles of an embodiment of the present invention preferably have a CV value of 2 to 80%. For example, the CV value may be 2, 5, 10, 15, 20, 25, 30, 35, 40, 45, 50, 55, 60, 65, 70, 75, or 80%, and may be in the range between the two values exemplified herein. The CV value can be calculated by the following formula:

$$CV\ value = standard\ deviation\ /\ average\ particle\ size \times 100$$

The method for producing polymer particles of an embodiment of the present invention is not particularly limited, but preferably includes a polymerization step of polymerizing a radically polymerizable compound in the presence of a chain transfer agent having a skeleton represented by general formula (23) and a polymerization initiator, and the polymerization step is preferably a polymerization step of polymerizing a radically polymerizable compound in an aqueous solvent. In addition, in the polymerization step, known compounds such as an emulsifier, a dispersant, an antioxidant and the like can be added as necessary.

In the polymerization step, the polymerization reaction may be carried out under static conditions or under stirring, but is preferably carried out under stirring. The polymerization temperature is preferably in the range of 30 to 100°C, more preferably in the range of 40 to 90°C, depending on the type and the amount of the monomer component, the type and the amount of the chain transfer agent and the polymerization initiator, and the like. The polymerization temperature may be constant from the start to the end of the polymerization, or may vary within the above range. The polymerization temperature may be controlled only by the reaction heat, or may be controlled by external heating and cooling. The polymerization time is not particularly limited, but is preferably 1 to 30 hours, more preferably in the range of 2 to 15 hours.

The polymer particles produced are preferably separated from the aqueous medium. The method for separating the particles from the aqueous medium is not particularly limited, and any known method can be appropriately applied. Examples of the separation method include filtration, reduced pressure filtration, centrifugation, spray drying, freeze drying, and evaporation-drying. The polymer particles separated from the aqueous medium can be further dried as necessary, and can be pulverized or crushed as necessary for use.

**Examples**

The present invention will be described in more detail below based on Examples, but the present invention is not limited to these.

(First Perspective)

The chain transfer agents CTA1 to 4 were synthesized by the following procedure.

- Synthesis of CTA1 (The chain transfer agent of Comparative Example 1)

A 200 ml two-necked eggplant flask was added with 27.58 ml (74.9 mmol) of 20 wt% solution of sodium ethoxide (manufactured by Tokyo Chemical Industry Co., Ltd.) and 100 ml of ethanol (manufactured by Junsei Chemical Co., Ltd.), and the flask was immersed in an ice bath. While stirring, 11.3 ml (74.9 mmol) of compound (4) (manufactured by Fujifilm Wako Pure Chemical Industries, Ltd.) was added dropwise to obtain solution (1). Another 500 ml two-necked eggplant flask was added with 20 ml of ethanol and 14.38 g (74.9 mmol) of compound (5) (manufactured by Tokyo Chemical Industry Co., Ltd.) and the flask was immersed in an ice bath. While maintaining the temperature in the flask at 10°C or lower, solution (1) was added dropwise, and the mixture was stirred for 3 hours after the dropwise addition. Then, 100 ml of water and 200 ml of dichloromethane (manufactured by Yoneyama Pharmaceutical Co., Ltd.) were added to extract the organic layer, which was then washed twice with 100 ml of saturated aqueous sodium chloride solution. Thereafter, the mixture was dehydrated using magnesium sulfate (manufactured by Yoneyama Chemical Industry Co., Ltd.), and the solvent was distilled off under reduced pressure to obtain CTA1 represented by formula (6).

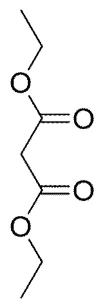

(4)

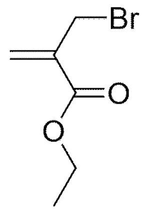

(5)

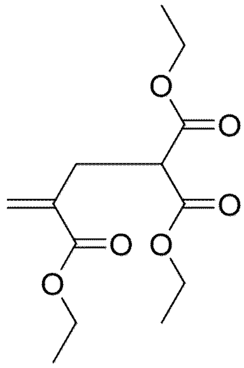

(6)

# CTA 1

- Synthesis of CTA2 (The chain transfer agent of Comparative Example 1)

A200 ml two-necked eggplant flask was added with 7.73 ml (21 mmol) of 20 wt% solution of sodium ethoxide and 10 ml of ethanol, and the flask was immersed in an ice bath. While stirring, 3.58 ml (21 mmol) of compound (7) (manufactured by Tokyo Chemical Industry Co., Ltd.) was added dropwise to obtain solution (2). Another 200 ml two-necked eggplant flask was added with 20 ml of ethanol and 2.92 ml (21 mmol) of compound (5), and the flask was immersed in an ice bath. While maintaining the temperature in the flask at 10°C or lower, solution (2) was added dropwise, and the mixture was stirred for 4 hours after the dropwise addition. Then, 100 ml of water and 100 ml of dichloromethane were added to extract the organic layer, which was then washed twice with 100 ml of saturated aqueous sodium chloride solution. Thereafter, the mixture was dehydrated using magnesium sulfate, and the solvent was distilled off under reduced pressure to collect the crude product of CTA2. The crude product of CTA2 was purified by silica gel column chromatography (Wakogel C400-HG manufactured by Fujifilm Wako Pure Chemical Industries, Ltd.) using hexane/ethyl acetate as a developing solvent to obtain CTA2 represented by formula (8).

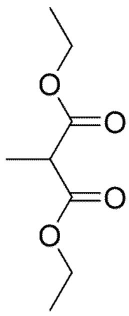

(7)

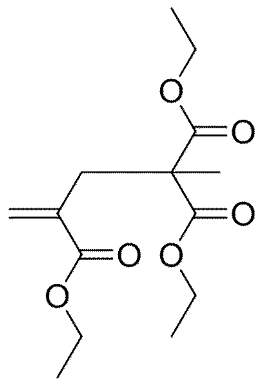

(8)

**CTA 2**

- Synthesis of CTA3 (The chain transfer agent of Example 1)

<Synthesis of compound (11)>

A 500 ml eggplant flask was added with 40 ml of acetonitrile (manufactured by Yoneyama Chemical Industries) and 10 ml of ethanol, and then 60 ml (564 mmol) of compound (9) (manufactured by Tokyo Chemical Industry Co., Ltd.), 31.6 ml (314 mmol) of compound (10) (manufactured by Fujifilm Wako Pure Chemical Industries, Ltd.), and 17.6 g (157 mmol) of 1,4-diazabicyclo[2.2.2]octane (DABCO) (manufactured by Tokyo Chemical Industry Co., Ltd.) were dissolved therein. The eggplant flask was immersed in an oil bath at 70°C and stirred for 24 hours. The mixture was then concentrated under reduced pressure at 50°C, and 200 ml of dichloromethane and 100 ml of water were added to extract the organic layer, which was then washed twice with 300 ml of saturated aqueous sodium chloride solution. The mixture was then dehydrated using magnesium sulfate, and the solvent was distilled off under reduced pressure to collect the crude product of compound (11). The collected crude product was purified by silica gel column chromatography (Wakogel C400-HG, manufactured by Fujifilm Wako Pure Chemical Industries, Ltd.) using hexane/ethyl acetate as a developing solvent to obtain compound (11).

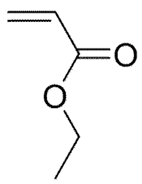

(9)

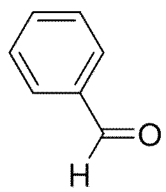

(10)

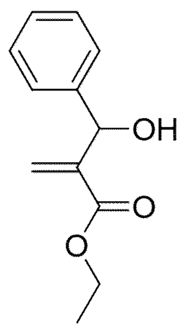

(11)

<Synthesis of compound (12)>

A 500 ml two-necked eggplant flask was added with 150 ml of dichloromethane and 15 g (75 mmol) of compound (11), and immersed in an ice bath. Next, 8.5 ml (90 mmol) of tribromophosphine (manufactured by Fujifilm Wako Pure Chemical Industries) was added dropwise, and after the completion of the dropwise addition, the temperature in the flask was kept at 15°C or lower, and stirred for 1 hour. Then, 50 ml of water was added dropwise, and while maintaining the temperature in the flask at 15°C or lower, 100 ml of saturated aqueous sodium bicarbonate was further poured in to extract the organic layer, and the organic layer was washed twice with 100 ml of saturated aqueous sodium chloride. Thereafter, the mixture was dehydrated using magnesium sulfate, and the solvent was distilled off under reduced pressure to obtain compound (12).

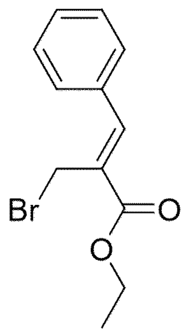

(12)

<Synthesis of CTA3>

A 100 ml two-necked eggplant flask was added with 40 ml of THF (ultra-dehydrated grade, manufactured by Kanto Chemical) and 1.52 g (38.1 mmol) of sodium hydride (manufactured by Fujifilm Wako Pure Chemical Industries) (60 wt% in oil), and immersed in an ice bath. Next, compound (4) was added dropwise while maintaining the temperature in the flask at 10°C or lower. After the completion of dropwise addition, the temperature of the flask was brought to 25°C and stirred for 30 minutes to obtain solution (3). Then, another 200 ml two-necked eggplant flask was added with 40 ml of THF and 10 g (37.1 mmol) of compound (12) and stirred, and further added with 1.5 g (44.6 mmol) of DABCO and immersed in an ice bath. While maintaining the temperature in the flask at 10°C or lower, solution (3) was added dropwise, and stirred for 3 hours after the completion of dropwise addition. Then, 200 ml of dichloromethane, 50 ml of water, and 100 ml of saturated sodium chloride solution were added to extract the organic layer, and the organic layer was washed twice with 100 ml of saturated sodium chloride solution. Then, the mixture was dehydrated using magnesium sulfate, and the solvent was distilled off under reduced pressure to obtain a crude product of CTA3. The crude product of CTA3 was purified by silica gel column chromatography (Wakogel C400-HG manufactured by Fujifilm Wako Pure Chemical Industries, Ltd.) using hexane/ethyl acetate as a developing solvent to obtain CTA3 represented by formula (13).

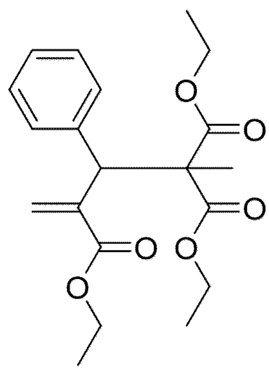

(13)

**CTA 3**

<Synthesis of CTA4>

<Synthesis of compound (15)>

In a 300 ml two-necked eggplant flask, 20 ml of acetonitrile, 16.38 ml (250 mmol) of compound (14) (manufactured by Tokyo Chemical Industry Co., Ltd.), 20.0 ml (198 mmol) of compound (10), and 22.43 g (200 mmol) of DABCO were dissolved. The eggplant flask was immersed in an oil bath at 50°C and stirred for 13 hours. Then, the mixture was concentrated under reduced pressure at 50°C, and 150 ml of dichloromethane and 80 ml of water were added to extract the organic layer, which was then washed twice with 150 ml of saturated aqueous sodium chloride solution. Then, the mixture was dehydrated using magnesium sulfate, and the solvent was distilled off under reduced pressure to collect the crude product of compound (15). The crude product of compound (15) was purified by silica gel column chromatography (Wakogel C400-HG manufactured by Fujifilm Wako Pure Chemical Industries) using hexane/ethyl acetate as a developing solvent, to obtain compound (15).

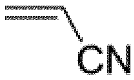

(14)

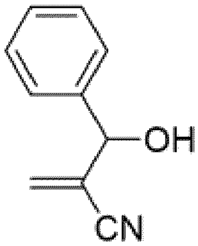

(15)

<Synthesis of compound (16)>

A 500 ml two-necked eggplant flask was added with 150 ml of dichloromethane and 5.0 g (31.4 mmol) of compound (14), and the flask was immersed in an ice bath. Next, 3.32 ml (35 mmol) of tribromophosphine was added dropwise, and after the completion of the dropwise addition, the temperature in the flask was maintained at 15°C or lower, and stirred for 1 hour. Then, 30 ml of water was added dropwise, and while maintaining the temperature in the flask at 15°C or lower, 50 ml of saturated aqueous sodium bicarbonate was further poured in to extract the organic layer, and the organic layer was washed twice with 70 ml of saturated aqueous sodium chloride. Thereafter, the mixture was dehydrated using magnesium sulfate, and the solvent was distilled off under reduced pressure to obtain compound (16).

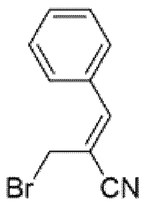

(16)

<Synthesis of CTA4>

A 50 ml two-necked eggplant flask was added with 10 ml of THF (ultra-dehydrated grade, manufactured by Kanto Chemical) and 0.72 g (18.0 mmol) of sodium hydride (60 wt% in oil), and immersed in an ice bath. Next, 3.01 ml (17.6 mmol) of compound (7) was added dropwise while maintaining the temperature in the flask at 10°C or lower. After the completion of the dropwise addition, the temperature in the flask was brought to 25°C, and stirred for 30 minutes to obtain solution (4). Then, another 300 ml two-necked eggplant flask was added with 190 ml of acetonitrile and 3.9 g (17.6 mmol) of compound (16), and stirred. Further, 1.974 g (17.6 mmol) of DABCO was added, and the flask was immersed in an ice bath. While maintaining the temperature in the flask at 10°C or lower, solution (4) was added dropwise, and after the completion of the dropwise addition, the flask was stirred for 3 hours. Thereafter, 200 ml of dichloromethane, 250 ml of water, and 100 ml of saturated sodium chloride solution were added to extract the organic layer, and the organic layer was washed twice with 200 ml of saturated sodium chloride solution. Then, the organic layer was dehydrated using magnesium sulfate, and the solvent was distilled off under reduced pressure to obtain a crude product of CTA4. The crude product of CTA4 was purified

by silica gel column chromatography using hexane/ethyl acetate as a developing solvent to obtain CTA4 represented by formula (17).

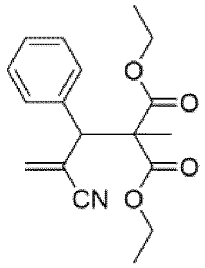

(17)

CTA4

Polymers were produced using the chain transfer agents CTA1 to 4 obtained above.

<Production of polymers>

(Example 1-1)

In a reaction apparatus equipped with a stirrer, a reflux condenser, a thermometer, and a nitrogen inlet tube, 100 parts by mass (0.78 mol) of butyl acrylate (BA), 0.32 parts by mass (0.87 mmol) of CTA3, and 130 parts by mass of ethyl acetate were charged, and the temperature was raised to 70°C while introducing nitrogen gas. Next, 0.1 parts by mass of 2,2'-azobisisobutyronitrile was added, and a polymerization reaction was carried out at 70°C for 3 hours under a nitrogen atmosphere to obtain a polymer.

(Example 1-2)

A polymer was obtained in the same manner as in Example 1-1, except that 0.28 parts by mass (0.87 mmol) of CTA4 was used instead of CTA3.

(Example 2-1)

A polymer was obtained in the same manner as in Example 1-1, except that the amount of CTA3 was 0.04 parts by mass (0.12 mmol).

(Example 2-2)

A polymer was obtained in the same manner as in Example 1-1, except that 0.01 parts by mass (0.03 mmol) of CTA4 was used instead of CTA3.

(Comparative Example 1)

A polymer was obtained in the same manner as in Example 1-1, except that 0.24 parts by mass (0.87 mmol) of CTA1 was used instead of CTA3.

(Comparative Example 2)

A polymer was obtained in the same manner as in Example 1-1, except that 0.25 parts by mass (0.87 mmol) of CTA2 was used instead of CTA3.

(Reference Example 1)

A polymer was obtained in the same manner as in Example 1-1, except that the chain transfer agent was not used.

The following evaluations were performed.

[Weight average molecular weight (Mw)]

The Mw of the polymers were determined by gel permeation chromatography (GPC) under the following conditions. The results are shown in Table 1.

- Measuring device: HLC-8320GPC (manufactured by Tosoh Corporation)
- GPC column configuration: The following four columns (all manufactured by Tosoh Corporation)

  (1) TSKgel HxL-H (guard column)
  (2) TSKgel GMHxL
  (3) TSKgel GMHxL
  (4) TSKgel G2500HxL

- Flow rate: 1.0 mL/min
- Column temperature: 40°C
- Sample concentration: 1.5% (w/v) (diluted by tetrahydrofuran)
- Mobile phase solvent: tetrahydrofuran
- Standard polystyrene equivalent

<Polymerization ratio>

The amount of residual monomer was measured under the following conditions using the internal standard method with gas chromatography. The amount of residual monomer was divided by the amount of butyl acrylate added to the reactor to calculate the polymerization ratio. The results are shown in Table 1.

- Measuring device: GC-2010Plus (manufactured by Shimadzu Corporation)
- Column: Rxi-5ms 15m, 0.25mmID, 0.25μm
- Column temperature: 50°C
- Heating program: 50°C (5min) -> 20°C/min -> 250°C (5min)
- FID temperature: 250°C
- Carrier gas: He

Table 1

| Table 1 | | | Example 1-1 | Example 1-2 | Example 2-1 | Example 2-2 | Comparative Example 1 | Comparative Example 2 | Reference Example 1 |
|---|---|---|---|---|---|---|---|---|---|
| Monomer | BA | parts by mass | 100 | | | | | | |
| Chain transfer agent | CTA1 | parts by mass | | | | | 0.24 | | |
| | CTA2 | parts by mass | | | | | | 0.25 | |
| | CTA3 | parts by mass | 0.32 | | 0.04 | | | | |
| | CTA4 | parts by mass | | 0.28 | | 0.01 | | | |
| Polymerization ratio[%] | | | 72 | 55 | 86 | 81 | 72 | 69 | 78 |
| Mw [x10000] | | | 39 | 15 | 83 | 88 | 89 | 93 | 120 |

Comparison of Examples 1-1, 1-2 with Comparative Examples 1, 2 showed that the molecular weight of the polymer can be controlled efficiently when the chain transfer agent of the present invention is used. Also, comparison of Examples 2-1, 2-2 with Comparative Examples 1, 2 showed that the amount of the chain transfer agent of the present invention used to produce polymers having similar molecular weight was small.

(Second perspective)

(Production Example 2-1)

A flask equipped with a stirrer, a nitrogen gas inlet tube, a thermometer, and a reflux condenser was charged with 100 parts by mass of n-butyl acrylate (BA), 16 parts by mass of the chain transfer agent CTA2-1 having the following structure, and 200 parts by mass of ethyl acetate, and the contents of the flask were heated to 70°C while introducing nitrogen gas into the flask. Next, 0.1 parts by mass of 2,2'-azobisisobutyronitrile (hereinafter referred to as "AIBN") was added. In order to maintain the temperature of the mixture at 70°C, heating and cooling were performed for 3 hours, and a solution containing Polymer 1 was obtained. After the reaction was completed, ethyl acetate was removed from the solution, and Polymer 1 was dissolved in tetrahydrofuran (THF) to prepare Solution 1 of Polymer 1/THF = 1 mg/mL. Then, Solution 1 was further diluted with matrix solution (2,5-dihydroxybenzoic acid (DHB)/tetrahydrofuran (THF) = 10 mg/mL) to a ratio of Solution 1:matrix solution = 1: 10 (mass ratio) to prepare an analytical solution containing Polymer 1.

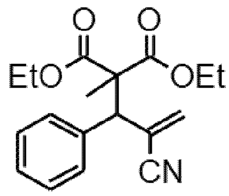

(25)

CTA2-1

<Analysis by MALDI/TOFMS>

The Polymer Solution 1 containing the obtained Polymer 1 was analyzed by a matrix-assisted laser desorption/ionization tandem time-of-flight mass spectrometer (MALDI/TOFMS) to obtain an MS spectrum. The measurement device, the measurement conditions and the like are as follows.

- Measuring Device

Device name: ultraflextreme
Manufacturer: Bruker Daltonics Inc.

- Measurement conditions

Ionization method: matrix-assisted laser desorption/ionization (MALDI)
Ionization mode: positive
Mass separation section: reflector mode

In the MS spectrum of Polymer 1, peaks were confirmed corresponding to the molecular weight as shown below.

Molecular weight = molecular weight of butyl acrylate (128) x n + molecular weight of the chain transfer agent CTA2-1 (315) + molecular weight of ionizing agent ($Na^+$ or $K^+$) (23 or 39)

In the MS spectrum of Polymer 1, peaks were confirmed corresponding to the molecular weight where n is an integer from 1 to 33 in the above formula. In other words, the presence of polymer chains having molecular weight of n butyl

acrylate monomer units and one molecule of the chain transfer agent CTA2-1 was confirmed. Note that n indicates the degree of polymerization of butyl acrylate (the repeating number of butyl acrylate monomer units).

In addition, in the MS spectrum of Polymer 1, peaks were confirmed that correspond to the molecular weight as shown below.

Molecular weight = molecular weight of butyl acrylate (128) x n + molecular weight of the portion derived from the chain transfer agent CTA2-1 shown in following formula (26) (142) + molecular weight of the terminal structure derived from the initiator (68) + molecular weight of --> the ionizing agent ($Na^+$ or $K^+$) (23 or 39)

In the MS spectrum of Polymer 1, peaks were confirmed corresponding to the molecular weight where n is an integer from 2 to 17 in the above formula. In other words, the presence of polymer chains having n butyl acrylate monomer units, a terminal structure derived from the chain transfer agent CTA2-1 after addition cleavage shown in following formula (26), and a terminal structure derived from the initiator shown in following formula (27) was confirmed. In addition, from this result, it can be understood that the chain transfer agent CTA2-1 is cleaved to produce a polymer chain having a terminal structure represented by formula (26) and a terminal structure derived from the radical shown in following formula (28).

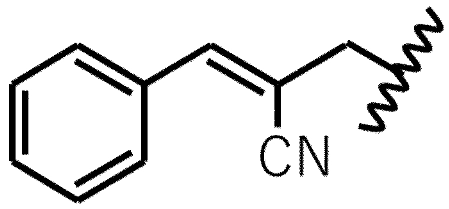

(26)

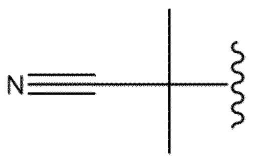

(27)

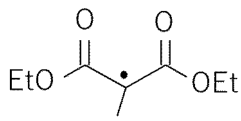

(28)

From the above, it was confirmed that in Production Example 2-1, a polymer chain was generated having the terminal structure represented by formula (26) derived from the above chain transfer agent CTA2-1 and the terminal structure represented by formula (27) derived from the initiator. From this, it is understood that a polymer chain having a molecular weight of n butyl acrylate monomer units and one molecule of the chain transfer agent CTA2-1 is a polymer chain having the terminal structure represented by formula (26) derived from the chain transfer agent CTA2-1 and the terminal structure represented by formula (29) derived from the chain transfer agent CTA2-1 shown below.

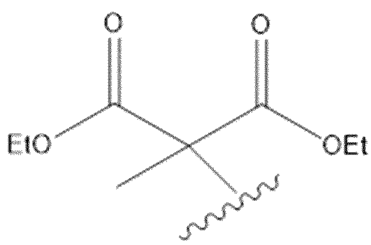

(29)

(Production Example 2-2)

Polymer Solution 2 containing Polymer 2 was prepared in the same manner as in Production Example 1, except that the chain transfer agent in Production Example 2-1 was changed to the chain transfer agent CTA2-3 having the following structure.

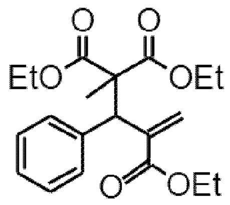

(30)

CTA2-3

<Analysis by MALDI/TOFMS>

In the same manner as Polymer Solution 1 containing Polymer 1, Polymer Solution 2 containing Polymer 2 was analyzed by MALDI/TOFMS to obtain an MS spectrum.

In the MS spectrum of Polymer 2, peaks were confirmed corresponding to the molecular weight as shown below.

Molecular weight = molecular weight of butyl acrylate (128) x n + molecular weight of the chain transfer agent CTA2-3 (362) + molecular weight of ionizing agent ($Na^+$ or $K^+$) (23 or 39)

In the MS spectrum of Polymer 2, peaks were confirmed corresponding to the molecular weight where n is an integer from 1 to 33 in the above formula.

In other words, the presence of polymer chains having molecular weight of n butyl acrylate monomer units and one molecule of the chain transfer agent CTA2-3 was confirmed. Note that n indicates the degree of polymerization of butyl acrylate (the repeating number of butyl acrylate monomer units).

In addition, in the MS spectrum of Polymer 2, peaks were confirmed that correspond to the molecular weight as shown below.

Molecular weight = molecular weight of butyl acrylate (128) x n + molecular weight of the portion derived from the chain transfer agent CTA2-3 shown in following formula (31) (189) + molecular weight of the terminal structure derived from the initiator (68) + molecular weight of the ionizing agent ($Na^+$ or $K^+$) (23 or 39)

In the MS spectrum of Polymer 2, peaks were confirmed corresponding to the molecular weight where n is an integer from 2 to 17 in the above formula. In other words, the presence of polymer chains having n butyl acrylate monomer units, a terminal structure derived from the chain transfer agent CTA2-3 after addition cleavage shown in following formula (31), and a terminal structure derived from the initiator shown in following formula (27) was confirmed. In addition, from this result, it can be understood that the chain transfer agent CTA2-3 is cleaved to produce a polymer chain having a terminal structure represented by formula (31) and a terminal structure derived from the radical shown in following formula (32).

From the above, it was confirmed that in Production Example 2-2, a polymer chain was generated having the terminal structure represented by following formula (31) derived from the chain transfer agent CTA2-3 and the terminal structure represented by formula (27) derived from the initiator. From this, it is understood that a polymer chain having a molecular weight of n butyl acrylate monomer units and one molecule of the chain transfer agent CTA2-3 is a polymer chain having the terminal structure represented by formula (31) derived from the chain transfer agent CTA2-3 and the terminal structure represented by following formula (32) derived from the chain transfer agent CTA2-3 shown below.

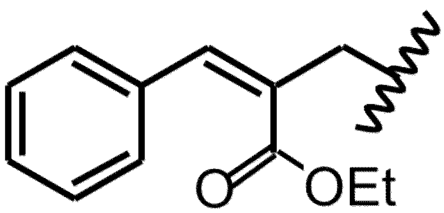

(31)

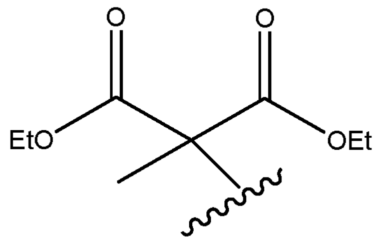

(32)

(Example 2-1)

A flask equipped with a stirrer, a nitrogen gas inlet tube, a thermometer, and a reflux condenser was charged with 40 parts by mass of n-butyl acrylate (BA), 55.8 parts by mass of 2-ethylhexyl acrylate (2EHA), 4 parts by mass of acrylic acid (AA), 0.2 parts by mass of 2-hydroxyethyl acrylate (2HEA), 0.09 parts by mass of CTA2-1, and 60 parts by mass of ethyl acetate, and the contents of the flask were heated to 70°C while introducing nitrogen gas into the flask. Next, 0.05 parts by mass of AIBN was added to the flask while stirring. In order to maintain the contents in the flask at 70°C, heating and cooling were performed for 2 hours and 30 minutes. Thereafter, the temperature was raised to 80°C, and a reflux reaction was performed for 2 hours to obtain Polymer (A-1). After the reaction was completed, the mixture was diluted with ethyl acetate to prepare a polymer solution containing Polymer (A-1) and having a solid concentration of 45% by mass. The Mw of the obtained (A-1) measured by GPC was 487,000. The corrosiveness was also confirmed as follows.

<Measurement of the weight average molecular weight (Mw)>

The weight average molecular weight (Mw) of the obtained polymer was determined by gel permeation chromatography (GPC) under the following conditions.

- Measuring device: HLC-8320GPC (manufactured by Tosoh Corporation)
- GPC column configuration: The following four columns (all manufactured by Tosoh Corporation)

    (1) TSKgel HxL-H (guard column)
    (2) TSKgel GMHxL
    (3) TSKgel GMHxL
    (4) TSKgel G2500HxL

- Flow rate: 1.0 mL/min
- Column temperature: 40°C
- Sample concentration: 1.5% (w/v) (diluted by tetrahydrofuran)
- Mobile phase solvent: tetrahydrofuran
- Standard polystyrene equivalent

<Polymerization ratio>

The amount of residual monomer was measured under the following conditions using the internal standard method with gas chromatography. The polymerization ratio was calculated assuming that all monomers other than those measured as residual monomers were involved in the polymerization.

- Measuring device: GC-2010Plus (manufactured by Shimadzu Corporation)
- Column: Rxi-5ms 15m, 0.25mmID, 0.25μm
- Column temperature: 50°C
- Heating program: 50°C (5min) -> 20°C/min -> 250°C (5min)
- FID temperature: 250°C
- Carrier gas: He

<Corrosiveness>

A polymer solution containing Polymer (A-1) was collected in a sample bottle, and a copper piece was put into the solution. The sample bottle was then heated at 150°C for 1 hour to evaporate the solvent in the polymer solution. The sample bottle was sealed and further heated at 150°C for 1 hour, after which the copper piece was visually inspected and

evaluated according to the following criteria:

Good: No change in the copper piece (not corrosive)
Bad: Discoloration of the copper piece was observed (corrosive)

(Example 2-2, 2-3, Comparative Example 2-1, Reference Example 2-1, 2-2)

Polymers A-2 to A-6 were produced in the same manner as in Example 2-1, except that the chain transfer agents used were as shown in Table 1, and the weight average molecular weight (Mw), Mw/Mn, and corrosiveness were evaluated. The structures of CTA2-1 and CTA2-3 are as described above, and CTA2-2 has a structure represented by a following formula. In Comparative Example 2-1, n-dodecyl mercaptan (NDM) was used, and in Reference Example 2, α-methylstyrene dimer (AMSD) was used. The results are shown in Table 1.

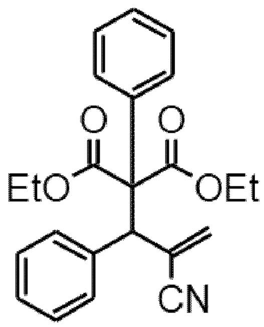

(33)

CTA2-2

Table 2-1

| Table 2-1 | Chain transfer agent | | Polymerization ratio | Mw[x10000] | Mw/Mn | Corrosiveness | Polymer |
|---|---|---|---|---|---|---|---|
| Example 2-1 | CTA2-1 | 0.09 parts by mass (0.3mmol) | 82.3% | 48.7 | 3.1 | Good | A-1 |
| Example 2-2 | CTA2-2 | 0.11 parts by mass (0.3mmol) | 80.4% | 43.9 | 2.9 | Good | A-2 |
| Example 2-3 | CTA2-3 | 0.28 parts by mass (0.8mmol) | 81.0% | 46.8 | 3.1 | Good | A-3 |
| Comparative Example 2-1 | NDM | 0.06 parts by mass (0.3mmol) | 84.4% | 44.5 | 2.8 | Bad | A-4 |
| Reference Example 2-1 | - | - | 85.2% | 92.3 | 4.9 | Good | A-5 |
| Reference Example 2-2 | AMSD | 0.07 parts by mass (0.3mmol) | 51.7% | 35.1 | 2.0 | Good | A-6 |

(Example 2-4)

The polymer solution containing Polymer A-1 (solid concentration 45% by mass) obtained in Example 2-1, Pencel D-135 (manufactured by Arakawa Chemical Industries Co., Ltd.) as a tackifier resin, and L-45 (manufactured by Soken Chemical Industries Co., Ltd.: isocyanate crosslinking agent) as a crosslinking agent were mixed in amounts such

that the solid content ratio was 100 parts by mass of Polymer A-1, 20 parts by mass of D-135, and 1.5 parts by mass of L-45, respectively, to obtain an adhesive composition.

The adhesive composition obtained above was left to stand on a polyethylene terephthalate (PET) film until bubbles were removed, and then coated with a doctor blade so that the thickness after drying would be 25 μm, and dried at 90°C for 3 minutes to remove the solvent, forming an adhesive layer. A release-treated PET film was attached to the surface of the adhesive layer opposite the surface in contact with the PET film. Thereafter, it was left to stand for 3 days at 40°C/dry conditions to allow aging, so that an adhesive sheet having an adhesive layer with a thickness of 25 μm was produced. The obtained adhesive sheet was used to perform the following evaluations.

<Adhesive force>

The adhesive sheet was cut to a size of 25 mm x 150 mm to prepare a test piece. The release-treated PET film of the obtained test piece was peeled off, and the exposed adhesive layer was attached to a stainless steel plate (SUS) and pressed by rolling 3 times with a 2 kg roller. After pressing, the test piece was left for 2 hours in an atmosphere of 23°C/50% RH, and then the short side of the test piece was pulled in the 180° direction at a peeling speed of 300 mm/min, and the adhesive force was measured.

<Holding power>

The adhesive sheet was cut to a size of 20 mm x 100 mm to prepare a test piece. The peeltreated PET film of the obtained test piece was peeled off, and the exposed adhesive layer was attached to a stainless steel plate (SUS) so that the adhesion area was 20 mm x 20 mm, and pressed by rolling 3 times with a 2 kg roller. Thereafter, the test piece was left to stand for 20 minutes in a 40°C/dry environment, and in the same environment, a load of 1 kg was applied in the shear direction to be tested, and the amount of displacement of the adhesive layer 1 hour after the start of load application was measured. When the test piece fell within 1 hour from the start of load application, the time from the start of load application to the fall was recorded.

<Probe tack>

The adhesive sheet was cut to a size of 20 mm x 150 mm, the release-treated PET film was peeled off, and the probe tack of the exposed adhesive layer was measured. The probe was made of SUS having a diameter of 5 mm, the contact time was 1 second, the probe speed was 1 cm/sec, and the load was 20 g.

(Examples 2-5 to 2-9, Comparative Example 2-2, 2-3, Reference Example 2-3, 2-4)

The adhesive sheets were produced and evaluated in the same manner as in Example 2-4, except that the type of polymers and the amount of crosslinking agents used were as shown in Table 2. The results are shown in Table 2.

Table 2-2

| Table 2-2 | Polymer [100 parts by mass] | amount of crosslinking agent [parts by mass] | Adhesive force [N/25mm] | Holding power [mm] | Probe tack [N] |
|---|---|---|---|---|---|
| Example 2-4 | A-1 | 1.5 | 14.4 | fell in 54 min | 3.0 |
| Example 2-5 | | 2.5 | 14.6 | 0.3 | 2.5 |
| Example 2-6 | A-2 | 1.5 | 14.1 | fell in 40 min | 2.7 |
| Example 2-7 | | 2.5 | 13.8 | 0.4 | 3.1 |
| Example 2-8 | A-3 | 1.5 | 13.9 | fell in 54 min | 3.4 |
| Example 2-9 | | 2.5 | 14.2 | 0.3 | 2.7 |
| Comparative Example 2-2 | A-4 | 1.5 | 15.1 | fell in 30 min | 2.7 |
| Comparative Example 2-3 | | 2.5 | 14.9 | 0.3 | 2.7 |
| Reference Example 2-3 | A-5 | 1.5 | 13.2 | 0.3 | 2.1 |
| Reference Example 2-4 | | 2.5 | 11.4 | 0.1 | 2.0 |

(Example 2-10)

A reaction apparatus equipped with a stirrer, a nitrogen gas inlet tube, a thermometer, and a reflux condenser was charged with 100 parts by mass of iso-butyl methacrylate (iBMA), 0.75 parts by mass of partially saponified polyvinyl alcohol as a dispersant, 0.16 parts by mass of CTA2-1, and 200 parts by mass of ion-exchanged water, and the contents of the flask were heated to 70°C while introducing nitrogen gas into the flask. Next, 1.5 parts by mass of lauroyl peroxide was added to the flask under stirring, and after the polymerization reaction was performed for 4 hours, the reaction system was cooled to obtain a dispersion of Polymer Particles 10. The obtained dispersion of Polymer Particles 10 was filtered and then dried to obtain Polymer Particles 10. The obtained polymer particles and the dispersion were evaluated by the following methods. The Mw of the Polymer Particles 10 was 164,000 and the particle diameter was 238 μm. The measurement methods for the weight average molecular weight (Mw) and Mw/Mn were the same as in Example 2-1.

<Polymerzation ratio (residual amount of iBMA in dispersion)>

The residual amount of iBMA (ppm) in the dispersion (polymerization solution after the completion of polymerization reaction) obtained in each Example was measured by gas chromatography, and the polymerization ratio was calculated. The measurement conditions for gas chromatography were as described above.

<Particle diameter>

Polymer Particles 10 were taken in the amount of 2 g, placed on a slide glass, and covered with a cover glass. Observation was performed from above the cover glass using a microscope (VHX-5000: manufactured by Keyence) (Figs. 1 and 2). Polymer Particles 10 were almost spherical. From the obtained observation image, the particle diameters of 10 polymer particles randomly chosen were measured, to calculate the average particle diameter.

<Corrosiveness>

After 3 g of Polymer Particles 10 were collected in a sample bottle, 5 g of ethyl acetate was added to dissolve the Polymer Particles 10. A copper piece was added to the sample bottle and heated at 150°C for 1 hour to evaporate the ethyl acetate. The sample bottle was sealed and further heated at 150°C for 1 hour, after which the copper piece was visually inspected and evaluated according to the following criteria:

Good: No change in the copper piece (not corrosive)
Bad: Discoloration of the copper piece was observed (corrosive)

(Examples 2-11 to 2-13, Comparative Example 2-4, Reference Example 2-5, 2-6)

The polymers were produced and evaluated in the same manner as in Example 2-10, except that the types and amounts of the crosslinking agents used were as shown in Table 3.

Table 2-3

| Table 2-3 | Chain transfer agent | | Polymerization ratio | Mw [x10000] | Mw/Mn | Average particle diameter[ μm] | Corrosiveness |
|---|---|---|---|---|---|---|---|
| Example 2-10 | CTA2-1 | 0.16 parts by mass (0.49 mmol) | 99% > | 16.4 | 1.9 | 238 | Good |
| Example 2-11 | CTA2-2 | 0.18 parts by mass (0.49 mmol) | 99% > | 15.5 | 2.1 | 254 | Good |
| Example 2-12 | CTA2-3 | 0.18 parts by mass (0.49mmol) | 99% > | 40.1 | 2.6 | 184 | Good |
| Example 2-13 | CTA2-3 | 0.47 parts by mass (1.3mmol) | 99% > | 16.9 | 2.3 | 201 | Good |

(continued)

| Table 2-3 | Chain transfer agent | | Polymerization ratio | Mw [x10000] | Mw/Mn | Average particle diameter[ $\mu$m] | Corrosiveness |
|---|---|---|---|---|---|---|---|
| Comparative Example 2-4 | NDM | 0.10 parts by mass (0.49 mmol) | 99% > | 15.8 | 2.0 | 248 | Bad |
| Reference Example 2-5 | AMSD | 0.12 parts by mass (0.49mmol) | 99% > | 26.1 | 2.2 | 177 | Good |
| Reference Example 2-6 | - | - | 99% > | 47.4 | 3.0 | 320 | Good |
| AMSD: $\alpha$ -methylstyrene dimer | | | | | | | |

From the above, it was shown that the polymer of the present invention is unlikely to cause problems such as corrosion and deterioration of adhesive properties, and the like. Furthermore, according to the production method of the present invention, it is possible to control the molecular weight with a sufficient polymerization ratio regardless of the type of radically polymerizable compound to be polymerized.

## Claims

1. A chain transfer agent represented by following general formula (1), wherein:

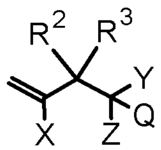

(1)

in general formula (1), X is a group selected from among a cyano group, an optionally substituted aryl group, an acyl group, a carboxy group, $COOR^1$, $C(O)NH_2$, $C(O)NHR^1$, $C(O)NR^1_2$, $SOR^1$, $SO_2R^1$, $SO_3R^1$, and $OR^1$;
Each of Y and Z is a group selected from among a cyano group, an optionally substituted aryl group, an acyl group, a carboxy group, $COOR^1$, $C(O)NH_2$, $C(O)NHR^1$, $C(O)NR^1_2$, $SOR^1$, $SO_2R^1$, $SO_3R^1$, $OR^1$, $OC(O)R^1$, $N(H)C(O)R^1$, and a halogen, or Y and Z are bonded to form an optionally substituted carbocyclic structure or an optionally substituted heterocyclic structure;
X, Y, and Z are the same or different;
$R^1$ is an optionally substituted alkyl group having 1 to 8 carbon atoms;
Q is hydrogen, an optionally substituted alkyl group having 1 to 6 carbon atoms or a phenyl group;
$R^2$ and $R^3$ are groups selected from among hydrogen, an alkyl group having 2 to 6 carbon atoms, an optionally substituted aryl group, and an optionally substituted heterocyclic structure;
$R^2$ and $R^3$ are the same or different; and
general formula (1) excludes a case where both $R^2$ and $R^3$ are hydrogen.

2. The chain transfer agent of Claim 1, wherein at least one of $R^2$ and $R^3$ is hydrogen.

3. The chain transfer agent of Claim 1, wherein at least one of $R^2$ and $R^3$ is a group selected from among an optionally substituted aryl group and an optionally substituted heterocyclic structure.

4. The chain transfer agent of Claim 1, wherein the chain transfer agent comprises a skeleton represented by general formula (23), wherein:

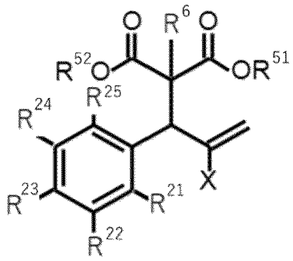

(23)

in general formula (23), $R^{21}$, $R^{22}$, $R^{23}$, $R^{24}$, and $R^{25}$ are groups selected from among hydrogen, an alkyl group having 1 to 8 carbon atoms, an alkoxy group having 1 to 8 carbon atoms, a cyano group, a carboxy group, and a halogen; $R^{21}$, $R^{22}$, $R^{21}$, $R^{24}$, and $R^{25}$ are either the same or different;
X is a group selected from among a cyano group, an optionally substituted aryl group, an acyl group, a carboxy group, $COOR^1$, $C(O)NH_2$, $C(O)NHR^1$, $C(O)NR^1{}_2$, $SOR^1$, $SO_2R^1$, $SO_3R^1$, and $OR^1$; $R^1$ is an optionally substituted alkyl group having 1 to 8 carbon atoms;
$R^{51}$ and $R^{52}$ are alkyl groups having 1 to 8 carbon atoms; $R^{51}$ and $R^{52}$ are the same or different;
$R^6$ is hydrogen, an optionally substituted alkyl group having 1 to 6 carbon atoms, or a phenyl group.

**5.** A production method of a polymer, comprising a polymerization step of polymerizing a radically polymerizable compound in the presence of the chain transfer agent of Claim 1 or 4.

**6.** The production method of Claim 5, wherein in the polymerization step, the radically polymerizable compound is polymerized in one or more of organic solvents selected from among ethyl acetate, ethyl methyl ketone, and toluene.

**7.** A production method of an adhesive composition, comprising a polymer production step and a crosslinking agent blending step, wherein:

in the polymer production step, a polymer is produced by the polymer production method of Claim 6, and
in the crosslinking agent blending step, at least one crosslinking agent selected from among an isocyanate crosslinking agent, an epoxy crosslinking agent, and a metal chelate crosslinking agent, is blended with the produced polymer.

**8.** The production method of Claim 5, wherein in the polymerization step, the radically polymerizable compound is polymerized in an aqueous medium.

**9.** A polymer containing a polymer chain having a group represented by following general formula (18) at an end thereof, wherein:

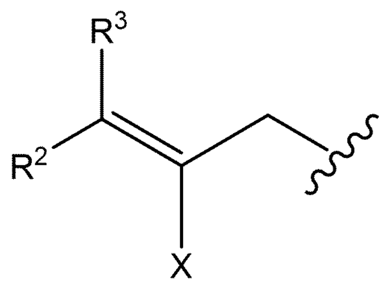

(18)

in general formula (18), X is a group selected from among a cyano group, an optionally substituted aryl group, an acyl group, a carboxy group, $COOR^1$, $C(O)NH_2$, $C(O)NHR^1$, $C(O)NR^1{}_2$, $SOR^1$, $SO_2R^1$, $SO_3R^1$, and $OR^1$; $R^1$ is an optionally substituted alkyl group having 1 to 8 carbon atoms;
$R^2$ and $R^3$ are groups selected from among hydrogen, an alkyl group having 2 to 6 carbon atoms, an optionally substituted aryl group, and an optionally substituted heterocyclic structure;
$R^2$ and $R^3$ are the same or different; and
general formula (1) excludes a case where both $R^2$ and $R^3$ are hydrogen.

**10.** The polymer of Claim 9, wherein the group represented by the above general formula (18) is represented by following

general formula (21), wherein:

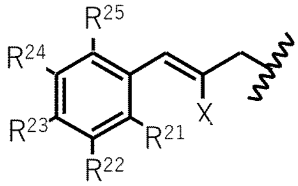

(21)

in general formula (21), $R^{21}$, $R^{22}$, $R^{23}$, $R^{24}$, and $R^{25}$ are groups selected from among hydrogen, an alkyl group having 1 to 8 carbon atoms, an alkoxy group having 1 to 8 carbon atoms, a cyano group, a carboxy group, and a halogen; $R^{21}$, $R^{22}$, $R^{21}$, $R^{24}$, and $R^{25}$ are the same or different;
X is a group selected from among a cyano group, an optionally substituted aryl group, an acyl group, a carboxy group, $COOR^1$, $C(O)NH_2$, $C(O)NHR^1$, $C(O)NR^1_2$, $SOR^1$, $SO_2R^1$, $SO_3R^1$, and $OR^1$;
$R^1$ is an optionally substituted alkyl group having 1 to 8 carbon atoms.

**11.** The polymer of Claim 9 or 10, wherein:

the polymer chain having a group represented by general formula (18) at an end thereof further has a group represented by following general formula (19) at an end thereof,

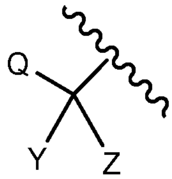

(19)

Each Y and Z is a group selected from among a cyano group, an optionally substituted aryl group, an acyl group, a carboxy group, $COOR^1$, $C(O)NH_2$, $C(O)NHR^1$, $C(O)NR^1_2$,$SOR^1$, $SO_2R^1$, $SO_3R^1$, $OR^1$, $OC(O)R^1$, $N(H)C(O)R^1$, and a halogen, or Y and Z are bonded to form an optionally substituted carbocyclic structure or an optionally substituted heterocyclic structure;
Y and Z are the same or different;
$R^1$ is an optionally substituted alkyl group having 1 to 8 carbon atoms;
Q is hydrogen, an optionally substituted alkyl group having 1 to 6 carbon atoms, or a phenyl group.

**12.** The polymer of Claim 11, wherein the group represented by the above general formula (19) is represented by following general formula (22), wherein:

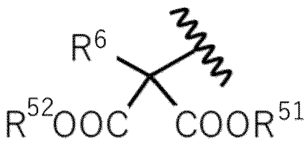

(22)

in general formula (22), $R^{51}$ and $R^{52}$ are alkyl groups having 1 to 8 carbon atoms; $R^{51}$ and $R^{52}$ are the same or different;
$R^6$ is hydrogen, an optionally substituted alkyl group having 1 to 6 carbon atoms, or a phenyl group.

**13.** The polymer of Claim 9 or 10, wherein a content ratio of sulfur derived from a thiol-based chain transfer agent is 100 ppm or less.

**14.** The polymer of Claim 9 or 10, wherein the polymer chain has a repeating monomer unit;
the repeating monomer unit has at least one group selected from among a carboxy group, a hydroxyl group, an amino group, and an amide group.

**15.** An adhesive composition comprising the polymer of Claim 14 and at least one crosslinking agent selected from among an isocyanate crosslinking agent, an epoxy crosslinking agent, and a metal chelate crosslinking agent.

**16.** A polymer particle comprising the polymer of Claim 9 or 10, wherein the polymer particle has an average particle diameter of 0.05 to 500 μm.

Fig. 1

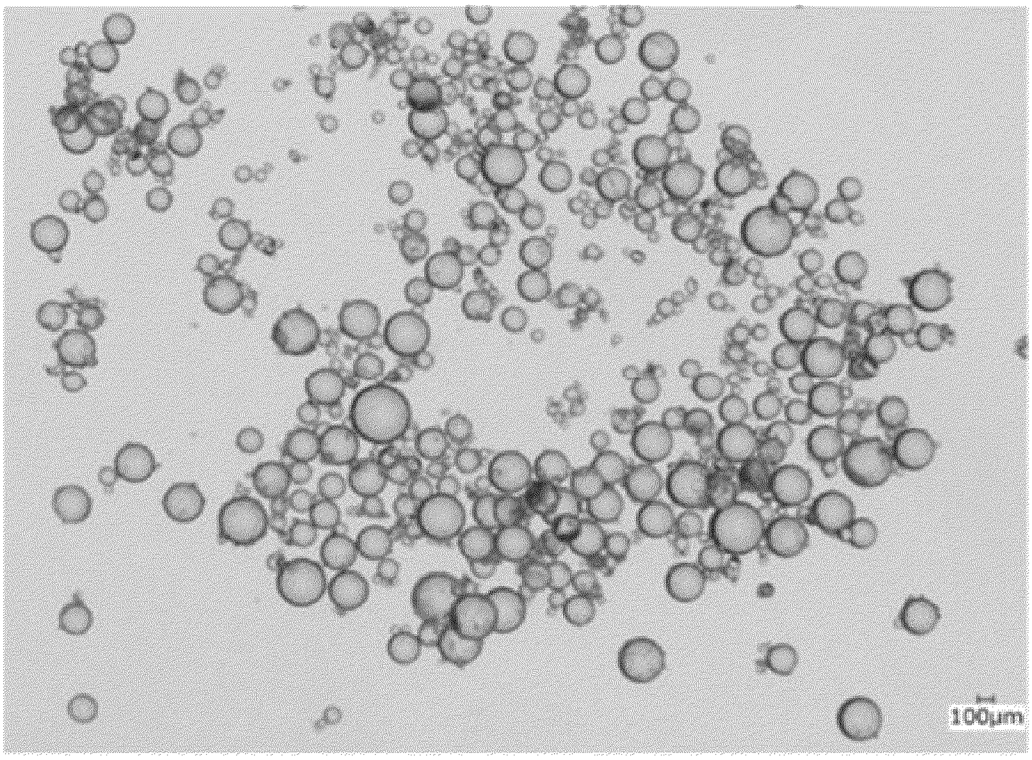


CTA-1: Example 10

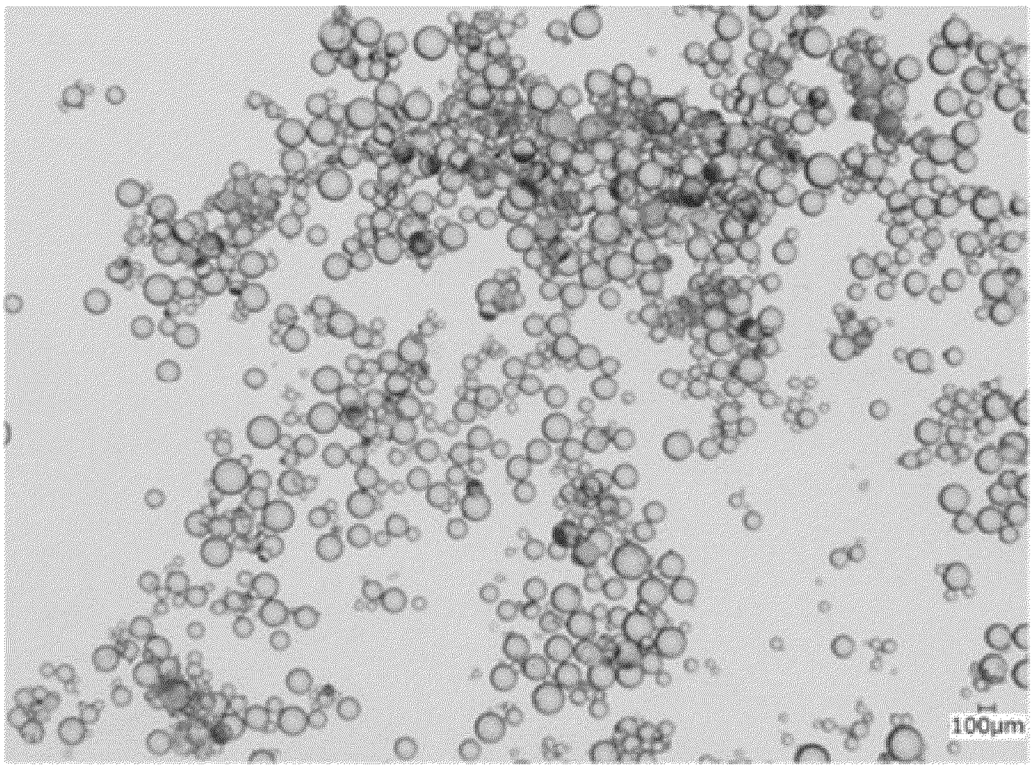


CTA-3: Example 12

Fig. 2

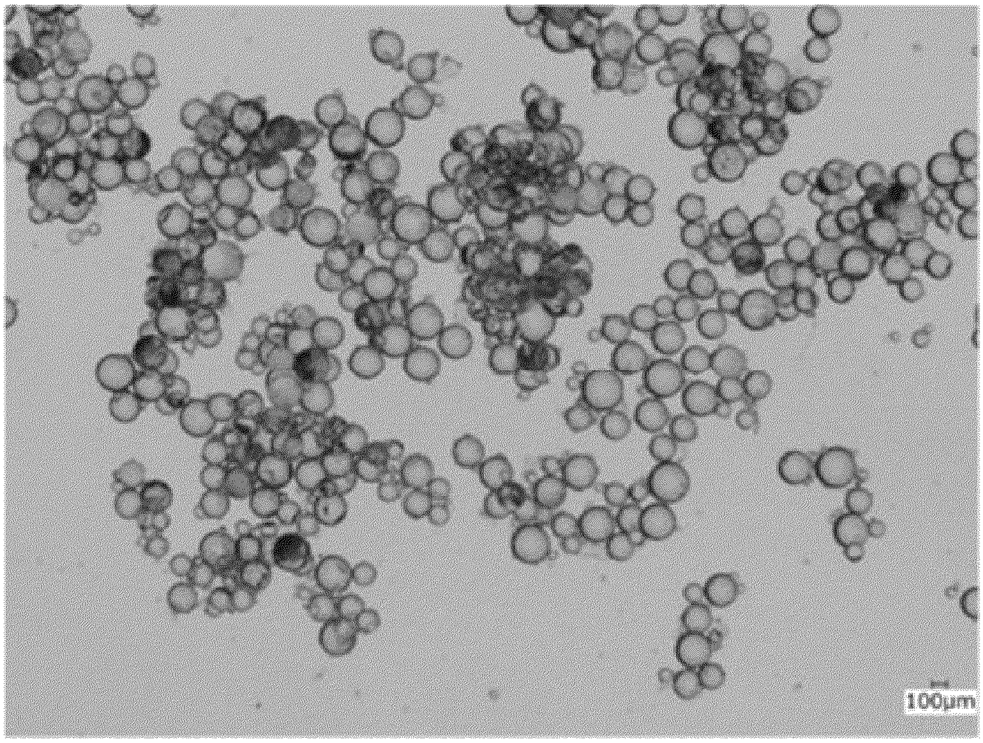


NDM: Comparative Example 4

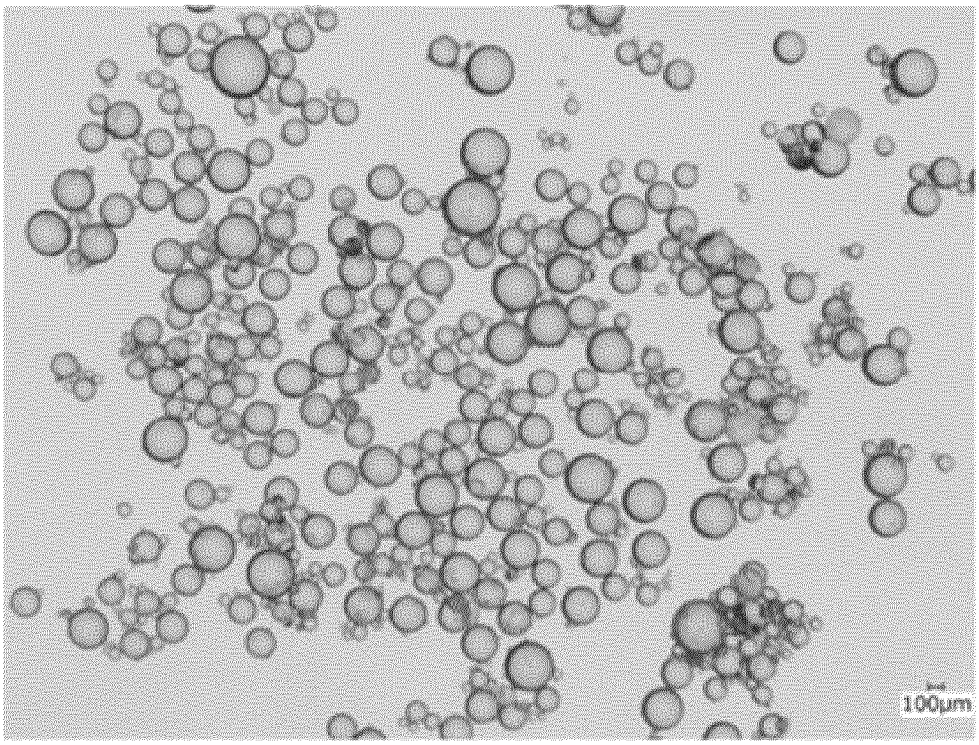


Without chain transfer agent: Reference Example 6

**INTERNATIONAL SEARCH REPORT**

International application No. **PCT/JP2023/012338**

**A. CLASSIFICATION OF SUBJECT MATTER**

***C08F 2/38***(2006.01)i; ***C08F 20/18***(2006.01)i
FI: C08F2/38; C08F20/18

According to International Patent Classification (IPC) or to both national classification and IPC

**B. FIELDS SEARCHED**

Minimum documentation searched (classification system followed by classification symbols)

C08F2/38; C08F20/18

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 09-510181 A (COMMONWEALTH SCIENTIFIC AND INDUSTRIAL RESEARCH ORGANISATION) 14 October 1997 (1997-10-14)<br>claims, p. 6, lines 6-9, p. 17, lines 23, 24, p. 18, lines 1-13, lines 20-25, examples | 1-2, 5-9, 11-16 |
| A | HUTSON, Lillian et al., Chain Transfer Activity of ω-Unsaturated Methacrylic Oligomers in Polymerizations of Methacrylic Monomers, Macromolecules, 18 May 2004, vol. 37, no. 12, pp. 4441-4452<br>entire text | 1-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 June 2023** | **13 June 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/012338**

| Patent document cited in search report | Publication date (day/month/year) | Patent family member(s) | Publication date (day/month/year) |
|---|---|---|---|
| JP 09-510181 A | 14 October 1997 | US 5773543 A<br>claims, column 1, lines 3-10, column 6, lines 57-59, column 6, line 65 to column 7, lines 14, column 7, lines 25-35, examples<br>WO 1995/012568 A1<br>EP 729449 A1<br>KR 10-1996-0705764 A<br>CN 1138320 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003321506 A
- JP 2004292428 A
- WO 2017022423 A